# FASCICULE DE BREVET EUROPEEN

(11) **EP 1 927 343 B1**
(45) Date de publication et mention de la délivrance du brevet: **29.02.2012**
(21) Numéro de dépôt: 07120372.3
(22) Date de dépôt: 09.11.2007
(51) Int. Cl.: A61K 8/35, A61K 8/49, A61Q 17/04

(54) **Composition photoprotectrice contenant un derivé de 1,3,5-triazine photosensible, un derivé du dibenzoylmethane, et une s-triazine siliciée et substituée par deux groupes aminobenzoates ou aminobenzamides**
Lichtschutzzusammensetzung, die ein lichtempfindliches 1,3,5-Triazinderivat, ein Dibenzoylderivat und ein mit zwei Aminobenzoat- oder Aminobenzamidgruppen substituiertes siliziumhaltiges s-Triazin enthält
Photoprotective composition containing a photosensitive 1,3,5-triazine derivative, a dibenzoylmethane derivative and a siliceous s-triazine substituted with two aminobenzoate or aminobenzamide groups

(30) Priorité: 28.11.2006 FR 0655164
(43) Date de publication de la demande: 04.06.2008
(73) Titulaire: L'Oréal, 75008 Paris (FR)
(72) Inventeur: Candau, Didier, 91570, BIEVRES (FR)
(74) Mandataire: Miszputen, Laurent

(56) Documents cités:
- EP-A- 0 841 341
- EP-A- 0 848 945
- EP-A- 0 933 376

## Description

L'invention se rapporte à une composition comprenant au moins, dans un support physiologiquement acceptable un système filtrant, caractérisée par le fait qu'elle comprend :
(a) au moins un filtre UV du type dérivé du dibenzoylméthane et
(b) au moins un filtre UV du type 1,3,5-triazine photosensible en présence d'un dérivé du dibenzoylméthane et
(c) au moins un composé s-triazine siliciée substituée par deux groupements aminobenzoates ou aminobenzamides de formule (I) ou l'une de ses formes tautomères que l'on définira plus en détail plus loin.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

A cet égard, une famille de filtres UV-A particulièrement intéressante est actuellement constituée par les dérivés du dibenzoylméthane, et notamment le 4-ter-butyl-4'-méthoxydibenzoyl méthane, qui présentent en effet un fort pouvoir d'absorption intrinsèque. Ces dérivés du dibenzoylméthane, qui sont maintenant des produits bien connus en soi à titre de filtres actifs dans les UV-A, sont notamment décrits dans les demandes de brevets français FR-A-2326405 et FR-A-2440933, ainsi que dans la demande de brevet européen EP-A-0114607 ; le 4-ter-butyl- 4'-méthoxydibenzoyl méthane est par ailleurs actuellement proposé à la vente sous la dénomination commerciale de "PARSOL 1789" par la Société HOFFMANN LAROCHE.

Les dérivés de 1,3,5-triazine sont particulièrement recherchés dans la cosmétique solaire du fait qu'ils sont fortement actifs dans l' UVB et même dans l'UV-A pour certains de ces composés selon la nature des substituants mis en jeu. Ils sont notamment décrits dans les demandes de brevet US 4367390, EP863145, EP517104, EP570838, EP507691, EP796851, EP775698, EP878469 et EP933376, et on connaît en particulier :
- la 2,4,6-tris[p-(2'-éthylhexyl-1'-oxycarbonyl)anilino]-1,3,5-triazine ou « Ethylhexyl Triazone » (nom INCl), vendue sous la dénomination commerciale « Uvinul T 150 » par la société BASF,
- la 2-[(p-(tertiobutylamido)anilino]-4,6-bis-[(p-(2'-éthylhexyl-1'-oxycarbonyl)anilino]-1,3,5-triazine ou « Diethylhexyl Butamido Triazone » (nom INCl) vendu sous le nom commercial « UVASORB HEB » par SIGMA 3V. Ils possèdent un fort pouvoir absorbant des UVB et il serait donc très intéressant de pouvoir les utiliser en association avec le 4-tert-butyl-4'-méthoxydibenzoylméthane cité ci-dessus dans le but d'obtenir des produits offrant une protection large et efficace dans l'ensemble du rayonnement UV.

Le document EP 848 945 A1 décrit l'effet photostabilisateur des dérivés siliciés à fonction benzalmalonate sur les mélanges de filtres de type dibenzoylmethane avec des dérivés de 1,3,5-triazine.

Le document EP 841 341 A1 divulgue des composés de s-triazine siliciés comme des filtres antisolaires dans des compositions cosmétiques photoprotectrices.

Toutefois, la Demanderesse a constaté que certains de ces dérivés de 1,3,5-triazine, lorsqu'ils sont en présence de 4-tert-butyl-4'-méthoxydibenzoylméthane sont photosensibles à savoir sous irradiation UV, ils présentent l'inconvénient de se dégrader chimiquement de façon importante. Dans ces conditions, l'association des deux filtres ne permet plus une protection solaire large prolongée de la peau et des cheveux.

La demanderesse a découvert de manière surprenante que l'introduction d'une s-triazine siliciée substituée par deux groupements aminobenzoates ou aminobenzamides de formule particulière (I) dans une composition contenant un dérivé de dibenzoylméthane, en particulier du 4-tert-butyl-4'-méthoxydibenzoylméthane, en association avec au moins un dérivé de 1,3,5-triazine photosensible, et en particulier avec la 2,4,6-tris[p-(2'-éthylhexyl-1'-oxycarbonyl)anilino]-1,3,5-triazine, permettait d'améliorer de façon tout à fait remarquable la photostabilité de ce dérivé de 1,3,5-triazine au sein de telles compositions, et donc l'efficacité globale de ces compositions.

Cette découverte est à la base de l'invention.

La présente invention a donc pour objet une composition comprenant au moins, dans un support physiologiquement acceptable un système filtrant, caractérisée par le fait qu'elle comprend :
(a) au moins un filtre UV du type dérivé du dibenzoylméthane et
(b) au moins un filtre UV du type 1,3,5-triazine photosensible en présence d'un dérivé du dibenzoylméthane et
(c) au moins un composé s-triazine siliciée substituée par deux groupements aminobenzoates ou aminobenzamides de formule (I) ou l'une de ses formes tautomères que l'on définira plus en détail plus loin.

Ainsi, selon la présente invention, on peut réaliser des compositions cosmétiques ou dermatologiques contenant du 4-tert-butyl-4'-méthoxydibenzoylméthane en association avec au moins un dérivé de 1,3,5-triazine photosensible, compositions dans lesquelles la concentration en dérivé de 1,3,5-triazine reste relativement constante même si ces compositions sont soumises à l'action de la lumière.

La présente invention a également pour objet un procédé pour améliorer la stabilité au rayonnement UV (photostabilité) d'un dérivé de 1,3,5-triazine photosensible en présence d'un dérivé de dibenzoylméthane; caractérisé par le fait qu'il consiste à ajouter à ladite association au moins un composé s-triazine siliciée substituée par deux groupements aminobenzoates ou aminobenzamides de formule (I) ou l'une de ses formes tautomères que l'on définira plus en détail plus loin.

La présente invention a encore pour objet l'utilisation d'au moins un composé s-triazine siliciée substituée par deux groupements aminobenzoates ou aminobenzamides de formule (I) ou l'une de ses formes tautomères que l'on définira plus en détail plus loin, dans une composition contenant au moins un dérivé du dibenzoylméthane et au moins un dérivé de 1,3,5-triazine photosensible en présence de celui-ci, dans le but d'améliorer la photostabilité dudit dérivé de 1,3,5-triazine.

D'autres caractéristiques, aspects et avantages de la présente invention apparaîtront à la lecture de la description détaillée qui va suivre.

Dans la suite de la présente description, on entend par « système filtrant les radiations UV » par un agent filtrant les radiations UV constitué soit d'un composé organique ou minéral unique filtrant les radiations UV soit un mélange de plusieurs composés organiques ou minéraux filtrant les radiations UV, par exemple mélange comprenant un filtre UVA et un filtre UVB.

Par « cosmétiquement acceptable », on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Par « filtre photosensible en présence d'un dérivé de dibenzoylméthane », on entend tout filtre susceptible de se dégrader chimiquement sous irradiation UV en présence d'un dérivé de dibenzoylméthane ; ce qui se traduit par une perte en quantité de filtre dans la composition et/ou une perte d'efficacité solaire après irradiation.

Selon la présente invention, les composés s-triazine siliciés de formule (I) seront utilisés dans une quantité suffisante permettant d'obtenir une amélioration notable et significative de la photostabilité du dérivé de 1,3,5-triazine dans une composition donnée contenant un dérivé de dibenzoylméthane. Cette quantité minimale en agent photostabilisant à mettre en oeuvre peut varier selon la quantité de triazine et de dibenzoylméthane présents au départ dans la composition et selon la nature du support cosmétiquement acceptable retenu pour la composition. Elle peut être déterminée sans aucune difficulté au moyen d'un test classique de mesure de photostabilité.

Parmi les dérivés du dibenzoylméthane conformes à l'invention, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert-butyl-4'-méthxydibenzoylméthane,
- le 2,4-diméthyl-4'-méthoxydibenzoylméthane,
- le 2,6-diméthyl-4-tert-butyl-4'-méthoxydibenzoylméthane.

Parmi les dérivés du dibenzoylméthane mentionnés ci-dessus, on utilisera en particulier le 4-isopropyl-dibenzoylméthane, vendu sous la dénomination de "EUSOLEX 8020" par la Société MERCK, et répondant à la formule suivante :

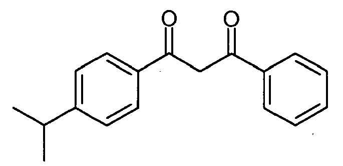

On préfère tout particulièrement mettre en oeuvre le 4-(ter.-butyl) 4'-méthoxy dibenzoylméthane ou Butyl Methoxy Dibenzoylmethane, proposé à la vente sous la dénomination commerciale de "PARSOL 1789" par la Société Roche Vitamins ; ce filtre répond à la formule suivante :

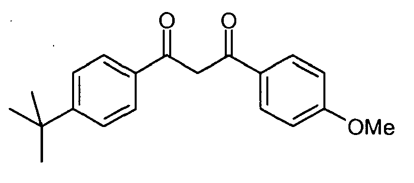

Le ou les dérivés du dibenzoylméthane peuvent être présents dans les compositions conformes à l'invention à des teneurs qui varient de préférence de 0,01 à 20% en poids et plus préférentiellement de 0,1 à 10% en poids et encore plus préférentiellement de 0,1 à 6% en poids par rapport au poids total de la composition.

Les composés s-trazines siliciées substituées par deux groupements aminobenzoates ou aminobenzamides conformes à l'invention répondent à la formule générale (I) suivante ou l'une de ses formes tautomères : 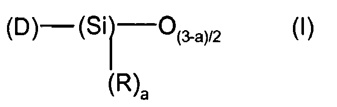 dans laquelle
- R, identiques ou différents représentent un radical alkyle en C₁-C₃₀, linéaire ou ramifié et éventuellement halogéné ou insaturé, un radical aryle en C₆-C₁₂, un radical alkoxy en C₁-C₁₀ ou le groupe triméthylsilyloxy ;
- a = 0 à 3;
- le groupe D désigne un composé s-triazine de formule (II) suivante : 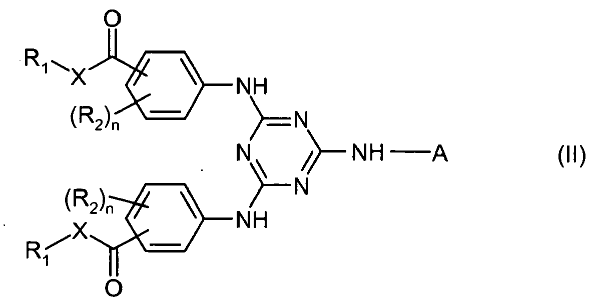
où
- X représente -O- ou -NR₃-, avec R₃ qui représente l'hydrogène ou un radical alkyle en C₁-C₅,
- R₁ représente un radical alkyle en C₁-C₂₀, linéaire ou ramifié et éventuellement insaturé et pouvant contenir un atome de silicium, un groupe cycloalkyle en C₅-C₂₀, éventuellement substitué par 1 à 3 radicaux alkyles en C₁-C₄, linéaires ou ramifiés, le groupe -(CH₂CHR₄-O)ₘR₅ ou le groupe -CH₂-CH(OH)-CH₂-O-R₆,
- R₄ représente l'hydrogène ou méthyle ; le groupement (C=O)XR1 pouvant être en position ortho, méta ou para du groupement amino,
- R₅ représente l'hydrogène ou un groupe alkyle en C1-C8,
- R₆ représentent l'hydrogène ou un groupe alkyle en C4-C8,
- m est un nombre entier allant de 2 à 20,
- n = 0 à 2,
- R₂, identiques ou différents, représentent un radical hydroxy, un radical alkyle en C₁-C₈, linéaire ou ramifié, un radical alcoxy en C1-C8, deux R2 adjacents d'un même noyau aromatique pouvant former ensemble un groupement alkylidène dioxy dans lequel le groupe alkylidène contient 1 ou 2 atomes de carbone,
- A est un radical divalent choisi parmi méthylène, -[CH(Si(CH₃)₃]-, éthylène ou un groupe répondant à l'une des formules (III), (IV) ou (V) suivantes :

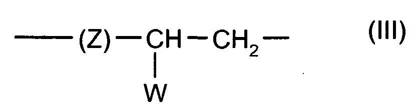

   -(Z)-CH=CH- (IV) 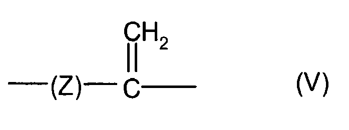
dans lesquelles :
- Z est un diradical alkylène en C₁-C₁₀, linéaire ou ramifié, saturé ou insaturé, éventuellement substitué par un radical hydroxyle ou des oxygènes et pouvant éventuellement contenir un groupement amino,
- W représente un atome d'hydrogène, un radical hydroxyle ou un radical alkyle en C₁-C₈, linéaire ou ramifié, saturé ou insaturé.

Il est à noter que les dérivés de formule (I) peuvent être utilisés sous leurs formes tautomères et plus particulièrement sous la forme tautomère de formule (I') suivante : 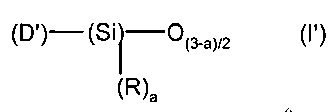 dans laquelle le groupe D' désigne un composé s-triazine de formule (II') suivante :

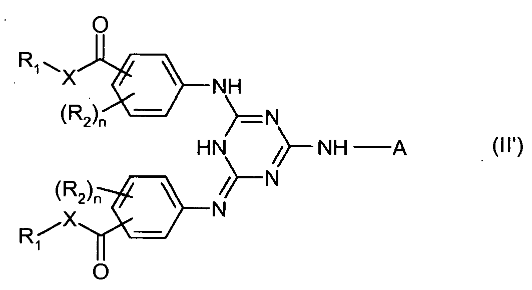

En plus des unités de formule -A-(Si)(R)ₐ(O)_{(3-a)/2}, l'organosiloxane peut comporter des unités de formule (R)_{b}-(Si)(O)_{(4-b)/2} dans lesquelles :
R a la même signification que dans la formule (I),
b = 1,2 ou 3.

Les dérivés de s-triazine préférentiels sont ceux pour lesquels dans la formule (II) ou (II') au moins l'une, et encore plus préférentiellement l'ensemble, des caractéristiques suivantes sont remplies :
R est méthyle,
a=1 ou 2,
X est O,
R₁ est un radical en C₄-C₅
n = 0 ,
le groupement (C=O)XR₁ est en position para vis-à-vis du groupement amino,
Z=-CH₂-,
W=H.

De manière préférée, les composés s-triazine de l'invention sont représentés par les formules (Ia), (Ib) ou (Ic) suivantes : 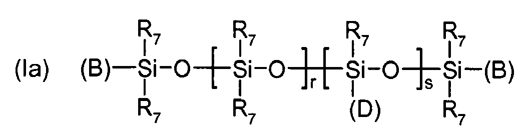 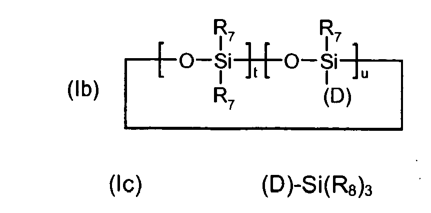 dans lesquelles :
- (D) répond à la formule (II) telle que définie ci-dessus,
- R₇, identiques ou différents, sont choisis parmi les radicaux alkyles linéaires ou ramifiés en C₁-C₂₀, phényle, 3,3,3-trifluoropropyle et triméthylsilyloxy ou le radical hydroxy,
- R₈, identiques ou différents, sont choisis parmi les radicaux alkyles et alcényles, linéaires ou ramifiés en C₁-C₂₀, les radicaux hydroxy ou phényle,
- (B), identiques ou différents sont choisis parmi les radicaux R₇ et le radical (D), r est un nombre entier compris entre 0 et 200 inclusivement,
- s est un nombre entier allant de 0 à 50 et si s = 0, au moins l'un des deux symboles (B) désigne (D),
- u est un nombre entier allant de 1 à 10,
- t est un nombre entier allant de 0 à 10, étant entendu que t + u est égal ou supérieur à 3 ainsi que leurs formes tautomères.

Les diorganosiloxanes linéaires de formule (Ia) sont particulièrement préférés.

Les diorganosiloxanes linéaires ou cycliques de formule (Ia) ou (Ib) rentrant dans le cadre de la présente invention, sont des oligomères ou polymères statistiques présentant de préférence au moins l'une, et encore plus préférentiellement l'ensemble, des caractéristiques suivantes :
- R7 est le radical méthyle ou le radical hydroxy
- B est préférentiellement méthyle (cas des composés linéaires de formule (Ia)),

A titre d'exemples de composés de formule (I) particulièrement préférés, on citera les composés de formules (a) à (m) suivantes ainsi que leurs formes tautomères :

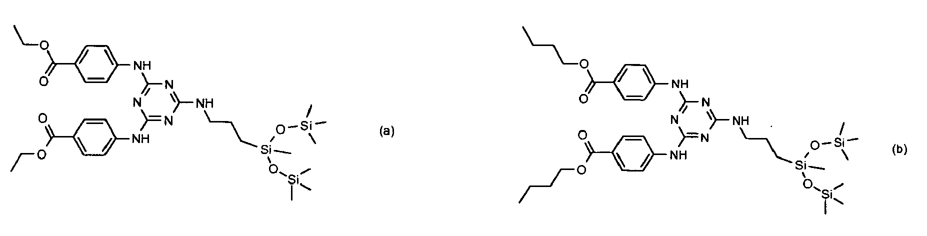

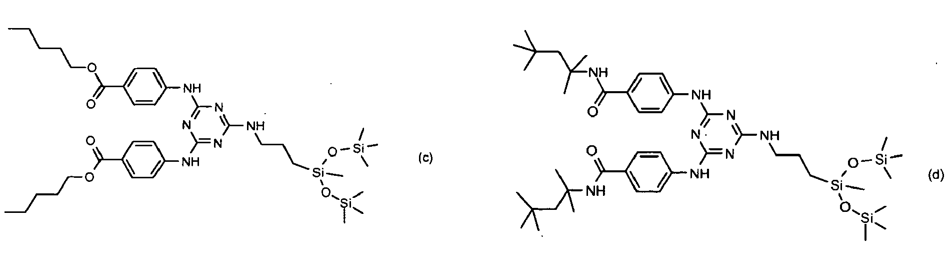

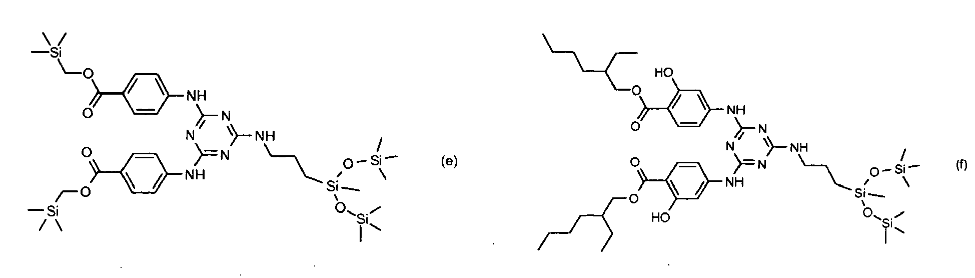

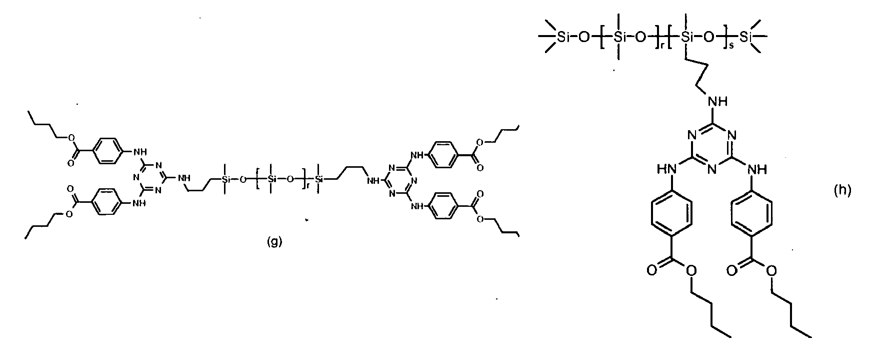

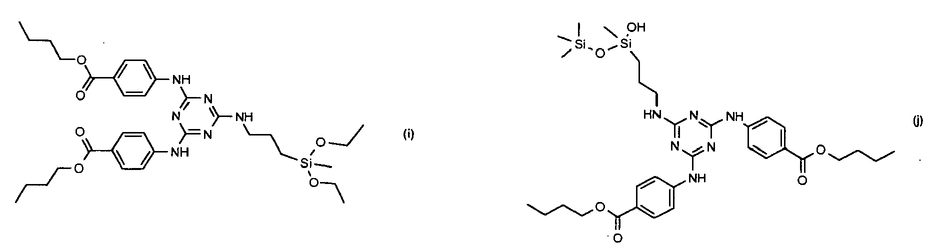

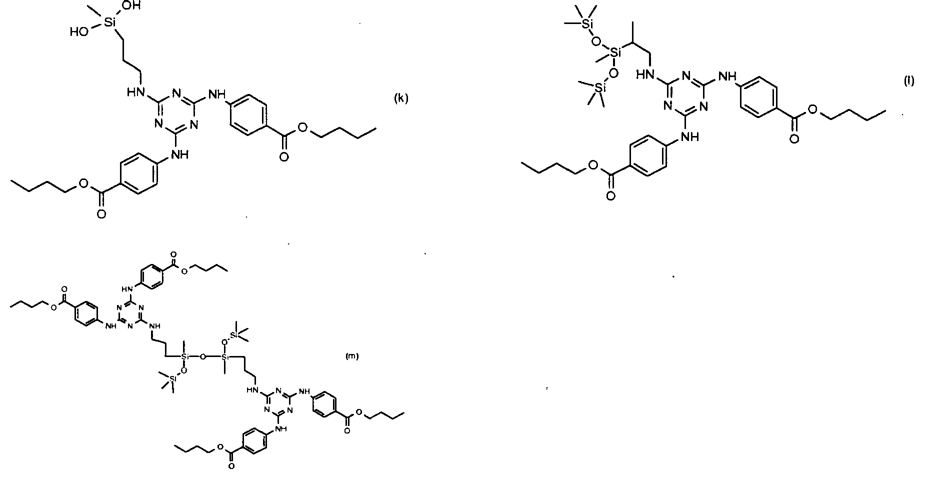

On utilisera plus particulièrement le composé 2,4-bis(4'-diylamino benzoate de n-butyle)-6-{[1,3,3,3-tétraméthyl-1-[(triméthylsilyl)oxy]disiloxanyl]propyl-3-ylamino}-s-triazine de structure (b) :

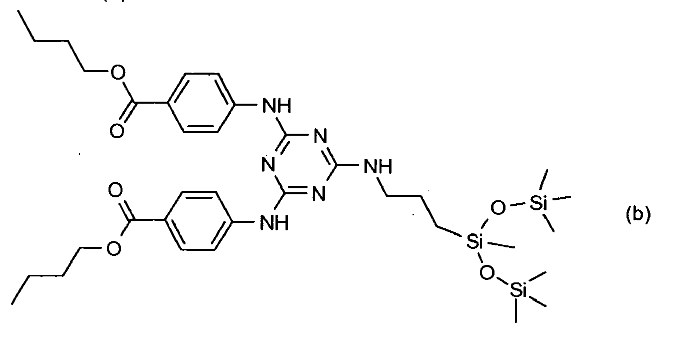

Les composés de formule (I) peuvent être obtenus selon le schéma réactionnel ci-dessous : 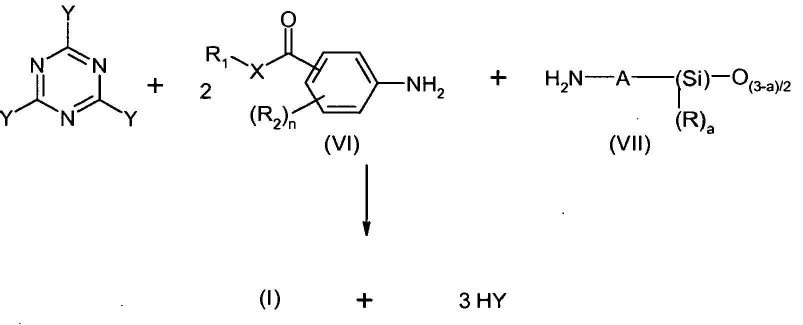 où R, R₁, R₂, A, n et a répondent aux définitions ci-dessus et Y représente un halogène, en particulier le chlore ou le brome.

L'ordre d'introduction des réactifs peut se faire indifféremment, 2 équivalents du dérivé de formule (VI) suivi d'un équivalent du dérivé de formule (VII) (voie 1) ou 1 équivalent du dérivé de formule (VII) suivi de 2 équivalents du dérivé de formule (VI) (Voie 2).

Les réactions ci-dessus peuvent être effectuées éventuellement en présence d'un solvant (par exemple : THF, acétone/eau pour la première étape ; toluène, xylène ou dichloro-1,2-éthane pour la deuxième étape), à une température comprise entre 0°C et 200°C, plus particulièrement entre 0°C et 20°C pour la première étape et entre 50 et 120°C pour la deuxième étape et en présence ou non d'une base captrice de l'acide formé (par exemple : bicarbonate de sodium, carbonate de sodium, soude aqueuse, triéthylamine ou pyridine). Elles peuvent être également réalisées en microondes en présence ou non d'un solvant (par exemple : toluène, xylène ou dichloro-1,2-éthane) ou en présence ou non de 10% de graphite, à une température de 50 à 150°C, à une puissance de 50-150 Watts pendant une durée de 10 à 30 minutes.

Lorsque a est égal à 1-3 et R est un alcoxy, les polymérisations des dérivés monomères alcoxysilanes peuvent être réalisées par des méthodes classiques de la chimie des silicones.

La préparation des dérivés aminés d'acide benzoïque de formule (VI) est décrite notamment dans FR 2151503. Comme dérivés aminés d'acide benzoïque convenant particulièrement bien à la préparation des composés selon l'invention, on peut citer le 4-amino benzoate de butyle et le 4-amino benzoate de pentyle.

Les silicones aminées de formule (VII) peuvent être obtenues chez Dow Corning Toray Silicone Co, Ldt telles celles de structure α,ω-diamino comme le BY16-853 (viscosité : 30; équivalent NH2 : 650) ou le BY16-853B (viscosité : 80; équivalent NH2 : 2200) ou celles de structure groupes pendants comme le BY16-828 (viscosité : 120; équivalent NH2 : 3500) ou le BY16-850 (viscosité : 1100; équivalent NH2 : 4000) ;

L'aminomethyltrimethylsilane vendu par la société Gelest et le bis(trimethylsilyl)methylamine (RN 134340-00-4).

Les composés s-triazines siliciées de formule (I) conformes à l'invention sont de préférence présents dans les compositions conformes à l'invention à des teneurs allant de 0,01 à 20% en poids et plus préférentiellement de 0,1 à 10 % et encore plus préférentiellement de 0,1 à 6% en poids par rapport au poids total de la composition.

Les filtres UV du type 1,3,5-triazine photyosansibles conformes à l'invention sont choisis de préférence parmi les dérivés de 1,3,5-triazine de formule (VIII) suivante : 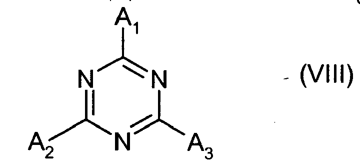 dans laquelle les radicaux A₁, A₂ et A₃, identiques ou différents sont choisis parmi les groupes de formules (IX) : 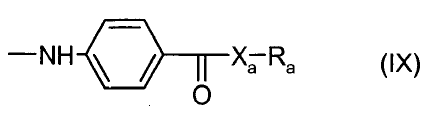 dans laquelle :
- Xₐ, identiques ou différents, représentent l'oxygène ou le radical -NH-;
- Rₐ, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (X), (XI) ou (XII) suivantes : 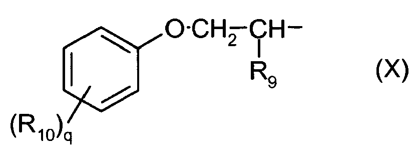 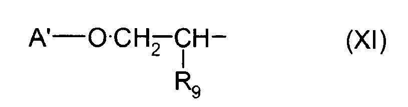 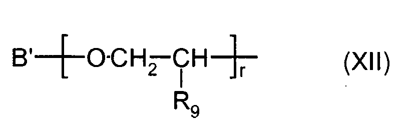
dans lesquelles :
- R₉ est l'hydrogène ou un radical méthyle;
- R₁₀ est un radical alkyle en C₁-C₉;
- q est un nombre entier allant de 0 à 3;
- r est un nombre entier allant de 1 à 10;
- A' est un radical alkyle en C₄-C₈ ou un radical cycloalkyle en C₅-C₈ ;
- B' est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄.

Une première famille plus particulièrement préférée de dérivés de 1,3,5-triazine, notamment décrite dans le document EP-A-0 517 104, est celle des 1,3,5-triazines répondant à la formule (VIII) dans laquelle les A₁, A₂ et A₃ sont de formule (IX) et présentent les caractéristiques suivantes :
- un des radicaux Xₐ-Rₐ représente le radical -NH-Rₐ avec Rₐ choisi parmi : un radical cycloalkyle en C₅-C₁₂ éventuellement substitué par un ou plusieurs radicaux alkyles en C₁-C₄; un radical de formule (X), (XI) ou (XII) ci-dessus dans lesquelles B' est un radical alkyle en C₁-C₄ et R₁₀ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (X), (XI) ou (XII) ci-dessus dans lesquelles B' est un radical alkyle en C₁-C₄ et R₁₀ est le radical méthyle.

Une deuxième famille plus particulièrement préférée de dérivés de 1,3,5-triazine, notamment décrite dans le document EP-A-0 570 838, est celle des 1,3,5-triazines répondant à la formule (VIII) dans laquelle les A₁, A₂ et A₃ sont de formule (IX) et présentant l'ensemble des caractéristiques suivantes :
- un ou deux Xₐ-Rₐ représente le radical -NH-Rₐ, avec Rₐ choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (X), (XI) ou (XII) ci-dessus dans lesquelles B' est un radical alkyle en C₁-C₄ et R₁₀ est le radical méthyle ;
- le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (X), (XI) ou (XII) ci-dessus dans lesquelles : B' est un radical alkyle en C₁-C₄ et R₁₀ est le radical méthyle.

Une 1,3,5-triazine particulièrement préférée de cette deuxième famille est la 2-[(p-(tertiobutylamido)anilino]-4,6-bis-[(p-(2'-éthylhexyl-1'-oxycarbonyl)anilino]-1,3,5-triazine ou « Diethylhexyl Butamido Triazone » vendue sous le nom commercial « UVASORB HEB » par SIGMA 3V et répondant à la formule suivante : 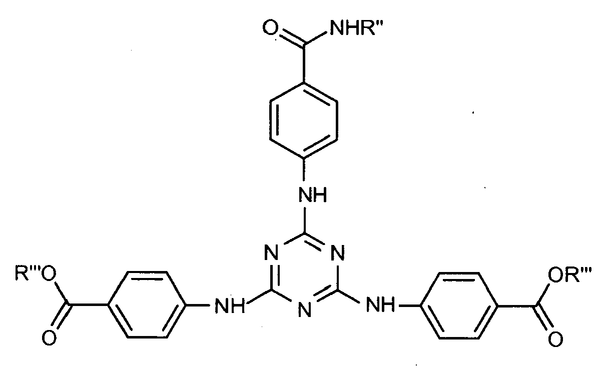 dans laquelle R"' désigne un radical éthyl-2 hexyle et R" désigne un radical tert-butyle.

Une troisième famille préférée de composés utilisables dans le cadre de la présente invention, et qui est notamment décrite dans le document US 4,724,137, est celle des 1,3,5-triazines répondant à la formule (VIII) dans laquelle les A₁, A₂ et A₃ sont de formule (IX) et présentent les caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

Une 1,3,5-triazine particulièrement préférée de cette troisième famille est la 2,4,6-tris[p-(2'-éthylhexyl-1'-oxycarbonyl)anilino]-1,3,5-triazine ou «Ethylhexyl Triazone » vendue notamment sous la dénomination commerciale de "UVINUL T 150" par la Société BASF et répond à la formule suivante : 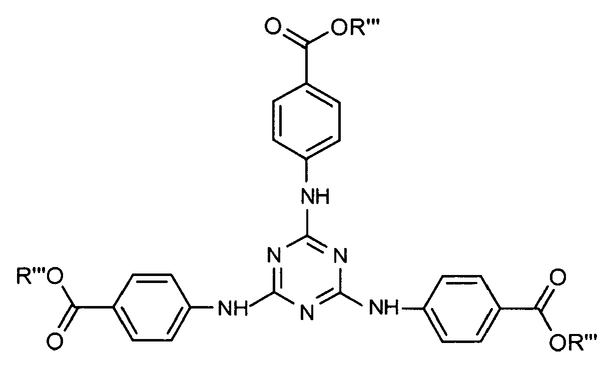 dans laquelle R"' désigne un radical 2-éthyl hexyle.

Les composés 1,3,5-triazine photosensibles sont de préférence présents dans les compositions conformes à l'invention à des teneurs allant de 0,01 à 20% en poids et plus préférentiellement de 0,1 à 10 % et encore plus préférentiellement de 0,1 à 6% en poids par rapport au poids total de la composition.

Les compositions selon l'invention sont généralement adaptées à une application topique sur la peau et comprend donc généralement un milieu physiologiquement acceptable, c'est-à-dire compatible avec la peau et/ou ses phanères (cheveux, cils, sourcils, ongles). Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

Les compositions conformes à l'invention peuvent comporter en plus d'autres filtres UV organiques ou inorganiques complémentaires actifs dans l'UVA et/ou l'UVB.

Bien entendu, l'homme de l'art veillera à choisir le ou les éventuels filtres complémentaires et/ou leurs quantités de manière telle que les propriétés avantageuses attachées intrinsèquement aux compositions conformes à l'invention ne soient pas, ou substantiellement pas, altérées par la ou les adjonctions envisagées.

Les agents photoprotecteurs organiques complémentaires sont notamment choisis parmi les anthranilates ; les dérivés cinnamiques ; les dérivés salicyliques, les dérivés du camphre ; les dérivés de la benzophénone ; les dérivés de β,β-diphénylacrylate ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples d'agents photoprotecteurs organiques complémentaires, on peut citer ceux désignés ci-dessous sous leur nom INCl :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
   - Diisopropyl Methylcinnamate,
Glyceryl Ethylhexanoate Dimethoxycinnamate

### Dérivés de β,β-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF, Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF, Benzophenone-12

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom « MEXORYL SL» par CHIMEX,
Terephthalylidene Dicamphor Sulfonic Acid fabriqué sous le nom « MEXORYL SX » par CHIMEX,
Camphor Benzalkonium Methosulfate fabriqué sous le nom « MEXORYL SO » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Polyorganosiloxane à fonctions benzalmalonate comme le Polysilicone-15 vendu sous la dénomination commerciale « PARSOL SLX » par HOFFMANN LA ROCHE

### Dérivés de 4,4-diarylbutadiène :

- 1,1-dicarboxy (2,2'-diméthyl-propyl)-4,4-diphénylbutadiène

Les agents photoprotecteurs organiques complémentaires préférentiels sont choisis parmi
Ethylhexyl Methoxycinnamate
Homosalate
Ethylhexyl Salicylate,
Octocrylene,
Phenylbenzimidazole Sulfonic Acid,
Terephthalylidene Dicamphor Sulfonic Acid
Disodium Phenyl Dibenzimidazole Tetra-sulfonate,
Benzophenone-3,
Benzophenone-4,
Benzophenone-5,
4-Methylbenzylidene camphor,
Polysilicone-15
1,1-dicarboxy (2,2'-diméthyl-propyl)-4,4-diphénylbutadiène
et leurs mélanges.

Les agents photoprotecteurs inorganiques complémentaires sont choisis parmi des pigments d'oxydes métalliques enrobés ou non (taille moyenne des particules primaires: généralement entre 5 nm et 100 nm, de préférence entre 10 nm et 50 nm) comme par exemple des pigments d'oxyde de titane (amorphe ou cristallisé sous forme rutile et/ou anatase), de fer, de zinc, de zirconium ou de cérium qui sont tous des agents photoprotecteurs UV bien connus en soi.

Les pigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

De façon connue, les silicones sont des polymères ou oligomères organo-siliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium.

Le terme "silicones" englobe également les silanes nécessaires à leur préparation, en particulier, les alkyl silanes.

Les silicones utilisées pour l'enrobage des pigments convenant à la présente invention sont de préférence choisies dans le groupe contenant les alkyl silanes, les polydialkylsiloxanes, et les polyalkylhydrogénosiloxanes. Plus préférentiellement encore, les silicones sont choisies dans le groupe contenant l'octyl triméthyl silane, les polydiméthylsiloxanes et les polyméthylhydro-génosiloxanes.

Bien entendu, les pigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Les pigments enrobés sont plus particulièrement des oxydes de titane enrobés :
- de silice tels que le produit "SUNVEIL" de la société IKEDA et le produit " Eusolex T-AVO" de la société MERCK
- de silice et d'oxyde de fer tels que le produit "SUNVEIL F" de la société IKEDA,
- de silice et d'alumine tels que les produits "MICROTITANIUM DIOXIDE MT 500 SA" et "MICROTITANIUM DIOXIDE MT 100 SA" de la société TAYCA, "TIOVEIL" de la société TIOXIDE, et « Mirasun TiW 60 » de la société Rhodia,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que le produit "MICROTITANIUM DIOXIDE MT 100 TV, MT 100 TX, MT 100 Z, MT-01 de la société TAYCA, les produits "Solaveil CT-10 W", "Solaveil CT 100" et "Solaveil CT 200" de la société UNIQEMA,
- de silice, d'alumine et d'acide alginique tel que le produit "MT-100 AQ" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS"de la société TAYCA,
- de silice, d'alumine, de stéarate d'aluminium et traités par une silicone tels que le produit "STT-30-DS" de la société TITAN KOGYO,
- de silice et traité par une silicone tel que le produit "UV-TITAN X 195" de la société KEMIRA, ou le produit SMT-100 WRS de la société TAYCA.
- d'alumine et traités par une silicone tels que les produits "TIPAQUE TTO-55 (S)" de la société ISHIHARA, ou "UV TITAN M 262" de la société KEMIRA,
   de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.

D'autres pigments d'oxyde de titane traités avec une silicone sont de préférence le TiO2 traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO2 traité par un polydiméthylsiloxane et dont la taille moyenne des particules élémentaires est de 21 nm tel que celui vendu sous la dénomination commerciale "70250 Cardre UF TiO2SI3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les pigments d'oxyde de zinc non enrobés, sont par exemple
- ceux commercialisés sous la dénomination "Z-cote" par la société Sunsmart ;
- ceux commercialisés sous la dénomination "Nanox" par la société Elementis ;
- ceux commercialisés sous la dénomination "Nanogard WCD 2025" par la société Nanophase Technologies ;

Les pigments d'oxyde de zinc enrobés sont par exemple
- ceux commercialisés sous la dénomination « Z-COTE HP1 » par la société SUNSMART (ZnO enrobé dimethicone) ;
- ceux commercialisés sous la dénomination "Oxide zinc CS-5" par la société Toshibi (ZnO enrobé par polymethylhydrogenesiloxane) ;
- ceux commercialisés sous la dénomination "Nanogard Zinc Oxide FN" par la société Nanophase Technologies (en dispersion à 40% dans le Finsolv TN, benzoate d'alcools en C12-C15) ;
- ceux commercialisés sous la dénomination "DAITOPERSION ZN-30" et "DAITOPERSION Zn-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD Ultrafine ZnO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "Escalol Z100" par la société ISP (ZnO traité alumine et dispersé dans le mélange methoxycinnamate d'ethylhexyle / copolymère PVP-hexadecene / methicone) ;
- ceux commercialisés sous la dénomination "Fuji ZnO-SMS-10" par la société Fuji Pigment (ZnO enrobé silice et polymethylsilsesquioxane) ;
- ceux commercialisés sous la dénomination "Nanox Gel TN" par la société Elementis (ZnO dispersé à 55% dans du benzoate d'alcools en C12-C15 avec polycondensat d'acide hydroxystéarique).

Les pigments d'oxyde de cérium non enrobé sont vendus par exemple sous la dénomination "COLLOIDAL CERIUM OXIDE" par la société RHONE POULENC.
Les nanopigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les pigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Les agents photoprotecteurs additionnels sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions selon l'invention peuvent également contenir des agents de bronzage et/ou de brunissage artificiels de la peau (agents autobronzants), et plus particulièrement la dihydroxyacétone (DHA). Ils sont présents de préférence dans des quantité allant 0,1 à 10% en poids par rapport au poids total de la composition.

Les compositions aqueuses conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire autre que les cires apolaires telles que définies précédemment ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras comme le benzoate d'alcools en C12-C15 vendu sous la dénomination commerciale « Finsolv TN » ou « Witconol TN » par la société WITCO, le le palmitate d'octyle, le lanolate d'isopropyle, les triglycérides dont ceux des acides caprique/caprylique, le dicaprylyl carbonate vendu sous la dénomination « Cetiol CC » par la société Cognis), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la cire de carnauba, la cire d'abeille, l'huile de ricin hydrogénée, les cires de polyéthylène et les cires de polyméthylène comme celle vendue sous la dénomination Cirebelle 303 par la société SASOL.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les Carbopols (Carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer /isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA: ammonium polyacryloyldimethyl taurate ou le SIMULGEL 800 commercialisé par la société SEPPIC (nom CTFA : sodium polyacryolyldimethyl taurate /polysorbate 80 / sorbitan oleate) ; les copolymères d'acide 2-acrylamido 2-méthylpropane sulfonique et d'hydroxyethyl acrylate comme le SIMULGEL NS et le SEPINOV EMT 10 commercialisés par la société SEPPIC ; les dérivés cellulosiques tels que l'hydroxyéthylcellulose ; les polysaccharides et notamment les gommes telles que la gomme de Xanthane ; et leurs mélanges.

Comme épaississants lipophiles, on peut citer les polymères synthétiques tels que les poly C₁₀-C₃₀ alkyl acrylates vendu sous la dénomination « INTELIMER IPA 13-1 » et « INTELIMER IPA 13-6 » par la société Landec ou encore les argiles modifiées telles que l'hectorite et ses dérivés, comme les produits commercialisés sous les noms de Bentone.

Parmi les actifs, on peut citer :
- les vitamines (A, C, E, K, PP...) et leurs dérivés ou précurseurs, seuls ou en mélanges,
- les agents anti-pollution et/ou agent anti-radicalaire ;
- les agents dépigmentants et/ou des agents pro-pigmentants ;
- les agents anti-glycation ;
- les agents apaisants,
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants;
- les agents tenseurs,
- les agents matifiants,
- les agents kératolytiques,
- les agents desquamants ;
- les agents hydratants ;
- les agents anti-inflammatoires ;
- les agents agissant sur le métabolisme énergétique des cellules,
- les agents répulsifs contre les insectes
- les antagonistes de substances P ou de CRGP.
- les agents anti-chute et/ou repousse des cheveux
- les agents anti-rides.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent être préparées selon les techniques bien connues de l'homme de l'art. Elles peuvent se présenter en particulier sous forme d'émulsion, simple ou complexe (H/E, E/H, H/E/H ou E/H/E) telle qu'une crème, un lait ou d'un gel crème ; sous la forme d'un gel aqueux ; sous la forme d'une lotion. Elles peuvent éventuellement être conditionnées en aérosol et se présenter sous forme de mousse ou de spray.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E). Les émulsions peuvent contenir également d'autres types de stabilisants des polymères gélifiants ou épaississants.

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les diméthicone copolyols tels que le mélange de cyclométhicone et de diméthicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostéarate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol.

Comme esters alkylés de polyol, on peut citer notamment les esters de polyéthylèneglycol comme le PEG-30 Dipolyhydroxystearate tel que le produit commercialisé sous le nom Arlacel P135 par la socité ICI ;
Comme esters de glycérol et/ou de sorbitan, on peut citer par exemple l'isostéarate de polyglycérol, tel que le produit commercialisé sous la dénomination Isolan Gl 34 par la société Goldschmidt ; l'isostéarate de sorbitan, tel que le produit commercialisé sous la dénomination Arlacel 987 par la société ICI ; l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) comme le mélange PEG-100 Stearate/ Glyceryl Stearate commercialisé par exemple par la société ICI sous la dénomination Arlacel 165 ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition auto-émulsionnante, comme décrit par exemple dans le document WO-A-92/06778.

Parmi les autres stabilisants d'émulsion, on utilisera plus particulièrement les polymères d'acide isophtalique ou d'acide sulfoisophtalique, et en particulier les copolymères de phtalate / sulfoisophtalate / glycol par exemple le copolymère de Diéthylèneglycol /Phtalate / Isophtalate / 1,4-cyclohexane-diméthanol (nom INCl : Polyester-5) vendu sous les dénominations "Eastman AQ polymer" (AQ35S, AQ38S, AQ55S, AQ48 Ultra) par la société Eastman Chemical.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).
Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin, des produits de protection solaire et des produits de maquillage.

Les compositions cosmétiques selon l'invention peuvent par exemple être utilisées comme produit de maquillage.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Des exemples concrets, mais nullement limitatifs, illustrant l'invention, vont maintenant être donnés.

### EXEMPLES DE SYNTHESE :

### EXEMPLE 1 : Préparation du 2,4-bis(4'-diylamino benzoate d'éthyle)-6-{[1,3,3,3-tétraméthyl-1-[(triméthylsilyl)oxy]disiloxanyl]propyl-3-ylamino}-s-triazine :

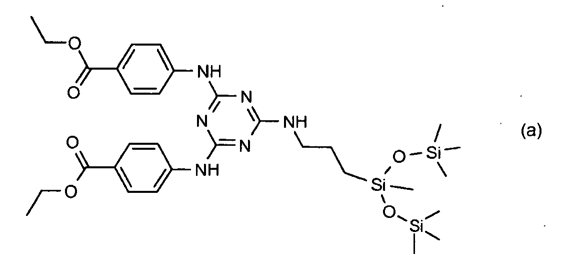

### Première étape : préparation de la 2,4-dichloro-6-{[1,3,3,3-tétraméthyl-1-[(triméthylsilyl)oxyldisiloxanyl]propyl-3-ylamino}-s-triazine:

A une solution de chlorure de cyanuryle (25 g, 0,135 mole) dans 250 ml d'acétone, on ajoute goutte à goutte à 0°C l'amino-1 [1,3,3,3-tétraméthyl-1-[(triméthylsilyl)oxy]disiloxanyl]-3-propane (41,7 g, 0,149 mole) et une solution de bicarbonate de sodium (11,4 g, 0,135 mole) dans 120 ml d'eau de telle sorte que le pH se situe entre 3 et 6,5. En fin d'introduction, le pH est de 6,5. L'agitation est ensuite maintenue 1 heure 30 minutes à 10°C, puis laissé à température du labo. Le précipité formé est filtré, lavé à l'eau, essoré et séché. On obtient 55,2 g (Rendement : 95%) du dérivé attendu sous forme d'une poudre blanche (Pf : 59°C).

### Deuxième étape : préparation du dérivé de l'exemple 1 :

Le mélange du produit précédent (2,1 g, 0,005 mole) et de para-amino benzoate d'éthyle (1,65 g, 0,01 mole) en suspension dans 20 ml de toluène est chauffé au reflux pendant 1 heure 30 minutes. On refroidit et ajoute à la résine obtenue de l'heptane chaud. Après trituration, filtration et séchage, on obtient 2,3 g (Rendement : 67%) du dérivé de l'exemple 1 sous forme d'une poudre blanche :
Pf : 106-108°C,
UV (Ethanol) : λmax=311 nm, E1% = 1147.

### EXEMPLE 2 : Préparation du 2,4-bis(4'-diylamino benzoate de n-butyle)-6-{[1,3,3,3-tétraméthyl-1-[(triméthylsilyl)oxy]disiloxanyl]propyl-3-ylamino}-s-triazine :

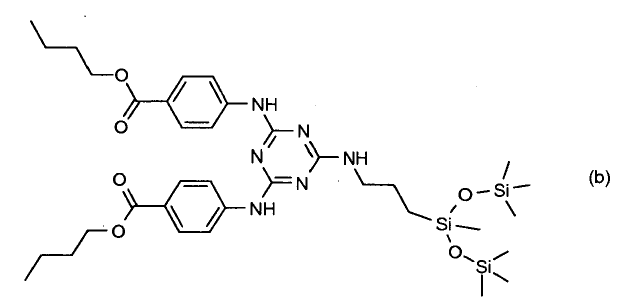

Sous barbotage d'azote, le mélange du produit de la première étape de l'exemple 1 (16,74 g, 0,0391 mole), du para-amino benzoate de n-butyle (15 g, 0,0776 mole) et du carbonate de potassium (5,36 g, 0,0388 mole) est mis en suspension dans 170 ml de toluène et est chauffé au reflux pendant 1 heure 20 minutes. On refroidit le mélange réactionnel et on y ajoute 150 ml de dichlorométhane. Les minéraux sont filtrés. Le filtrat est lavé à l'eau bicarbonatée puis 2 fois à l'eau. On obtient après séchage de la phase organique et évaporation des solvants une poudre blanche. Après recristallisation dans un mélange EtOAc/Heptane 1 :15, on obtient 20,1 g (Rendement: 69%) du dérivé de l'exemple 2 sous forme d'une poudre blanche :
Pf : 111-113°C,
UV (Ethanol) : λmax = 312 nm , E1% = 1055.

### EXEMPLE 3 : Préparation du 2,4-bis(4'-diylamino benzoate de n-pentyle)-6-{[1,3,3,3-tétraméthyl-1-[(triméthylsilyl)oxy]disiloxanyl]propyl-3-ylamino}-s-triazine :

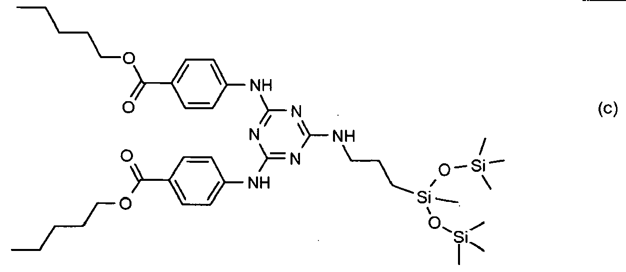

Dans un micro ondes CEM Discover, on chauffe pendant 20 minutes à une température de 115°C et sous une puissance de 150 Watt le mélange du produit de la première étape de l'exemple 1 (1 g, 2,3x10-3 mole), du para-amino benzoate de n-pentyle (0,97 g, 4,6x10-3 mole) et du bicarbonate de sodium (0,39g, 4,6x10-3 mole) dans 15 ml de toluène. On ajoute au mélange réactionnel du dichlorométhane et lave avec une solution saturée de chlorure de sodium puis 2 fois à l'eau. On obtient après séchage de la phase organique et évaporation des solvants une huile transparente. Après purification sur colonne de silice (éluant : Heptane/EtOAc 85 :15), on récupère les fractions propres du dérivé de l'exemple 3 (0,9 g, Rendement : 50%) sous forme d'une poudre blanche : UV (Ethanol) : λmax = 312 nm , E1% = 1008.

### EXEMPLE 4 : Préparation du 2,4-bis[4'-diylamino benzamide de (1,1,3,3-tétraméthylbutyl)]-6-{[1,3,3,3-tétraméthyl-1-[(triméthylsilyl)oxy]disiloxanyl]propyl-3-ylamino}-s-triazine :

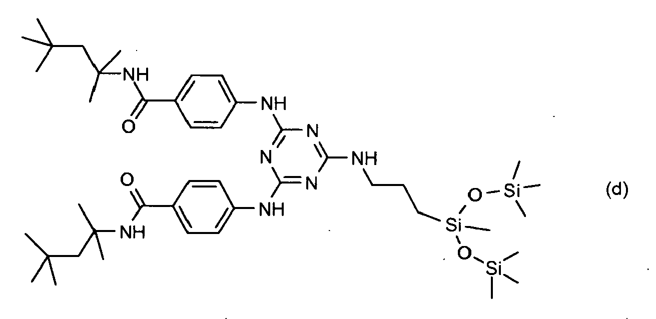

### Première étape : préparation du 4-nitro-N-(tert-octyl) benzamide :

Dans un réacteur, on introduit de la tert-octyl amine (51,7 g, 0,4 mole) et de la triéthylamine (61,2 ml, 0,44 mole) dans 260 ml de dichloroéthane. On chauffe à 70°C puis on ajoute en 50 minutes le 4-nitrobenzoyl chloride (77,9 g, 0,42 mole) par petites portions. On chauffe au reflux pendant 4 heures. On verse le mélange réactionnel sur de l'eau glacée ; on extrait au dichlorométhane, sèche et évapore le solvant. Le précipité beige obtenu est recristallisé dans un mélange d'éther isopropylique et d'éthanol (rapport 10 :1). Après séchage sous vide, on obtient 84,6 g (Rendement 76%) du 4-nitro-N-(tert-octyl) benzamide sous forme d'une poudre blanc cassé et utilisée telle quelle dans l'étape suivante.

### Deuxième étape : préparation du 4-amino-N-(tert-octyl) benzamide :

Dans un hydrogénateur de 500 ml, du 4-nitro-N-(tert-octyl) benzamide (30 g, 0,108 mole) dissout dans 200 ml d'acétate d'éthyle est hydrogéné en présence de 4,8 g de palladium à 10% sur charbon à 50% d'eau comme catalyseur (pression d'hydrogène : 8-10 bars) à une température de 70-75°C pendant 1 heure et 15 minutes. Après filtration, concentration du solvant et séchage sous vide, on obtient 20,4 g (Rendement : 76%) de 4-amino-N-(tert-octyl) benzamide sous forme d'une poudre jaune clair et utilisée telle quelle dans l'étape suivante.

### Troisième étape : préparation du dérivé de l'exemple 4 :

Dans un micro ondes CEM Discover, on chauffe pendant 20 minutes à une température de 115°C et sous une puissance de 150 Watt le mélange du produit de la première étape de l'exemple 1 (1 g, 2,3x10-3 mole), du produit de l'étape précédente (1,16 g, 4,6x10-3 mole) et du bicarbonate de sodium (0,39g, 4,6x10-3 mole) dans 10 ml de toluène sec. On ajoute au mélange réactionnel du dichlorométhane et lave avec une solution saturée de chlorure de sodium puis 2 fois à l'eau. On obtient après séchage de la phase organique et évaporation des solvants une huile jaune clair. Après purification sur colonne de silice (éluant : Heptane/EtOAc 70 :30), on récupère les fractions propres du dérivé de l'exemple 3 (0,9 g, Rendement : 45%) sous forme de paillettes blanches : UV (Ethanol) : λmax = 302 nm , E1% = 775.

### EXEMPLE 5 : Préparation du 2,4-bis(4'-diylamino benzoate de méthyltriméthylsilyl)-6-{[1,3,3,3-tétraméthyl-1-[(triméthylsilyl)oxy]disiloxanyl]propyl-3-ylamino}-s-triazine :

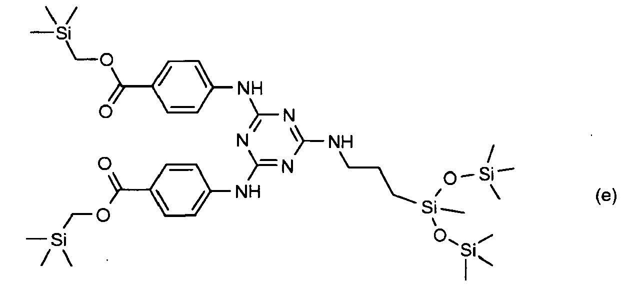

### Première étape : préparation du 4-aminobenzoate de méthyltriméthylsilyle :

Dans un réacteur, on ajoute goutte à goutte à 80°C du chlorométhyltriméthylsilyle (38,5 g, 0,314 mole) au mélange hétérogène du sel de potassium de l'acide para-amino benzoïque (50 g, 0,285 mole) dans 350 ml de DMF. On chauffe au reflux pendant 3 heures. Après refroidissement, on filtre les sels et on évapore le DMF. Le résidu est repris dans du dichlorométhane, séché et le solvant évaporé. L'huile obtenue est purifiée par distillation. On récupère les fractions qui distillent à 189°C sous un vide de 0,6 mbar. L'huile cristallise. On obtient 50,4 g (Rendement : 79%) du dérivé de l'exemple 5 sous forme d'une poudre blanche et utilisée telle quelle dans l'étape suivante.

### Deuxième étape : préparation du dérivé de l'exemple 5 :

Sous barbotage d'azote, le mélange du produit de la première étape de l'exemple 1 (2,1 g, 4,9x10-3 mole) et du dérivé de l'étape précédente (2,19 g, 9,8x10-3 mole) dans 40 ml de toluène est chauffé au reflux pendant 5 heures. On refroidit et on évapore le solvant. Le résidu est repris dans du dichlorométhane, séché et le solvant évaporé. On obtient 3 g (Rendement : 76%) du dérivé de l'exemple 5 sous forme d'une gomme jaune pâle : UV (Ethanol) : λmax = 311 nm , E1% = 907.

### EXEMPLE 6 : Préparation du 2,4-bis(2'-hydroxy-4'-diylamino benzoate d'éthyl-2-hexyle)-6-{[1,3,3,3-tétraméthyl-1-[(triméthylsilyl)oxy]disiloxanyl]propyl-3-ylamino}-s-triazine :

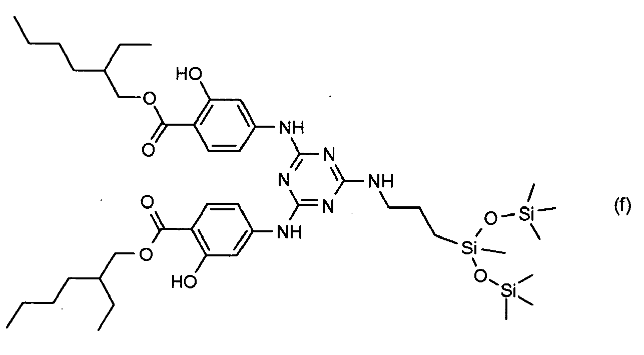

Sous barbotage d'azote, un mélange de 2-hydroxy-4-aminobenzoate d'éthyl-2-hexyle (1,4 g, 5,57x10-3 mole) et du produit de la première étape de l'exemple 1 (1,19 g, 2,78x10-3 mole) dans 10 ml de toluène est chauffé au reflux pendant 5 heures. On refroidit et on évapore le solvant. Le résidu est chromatographié sur colonne de silice (éluant : Heptane/EtOAc 9/1). On obtient 1,58 g (Rendement: 64%) des fractions propres du dérivé de l'exemple 6 sous forme d'une pâte blanche :
UV (Ethanol) : λ = 300 nm, E1% = 480
λmax = 325 nm , E1% = 709.

### EXEMPLE 7 : Préparation du dérivé statistique de formule (Ia,III) : R₁ = n-butyl, X = O, n = 0, B = A, W = H, Z = CH2, R₇ = CH₃, s = 0, r = 8,1 :

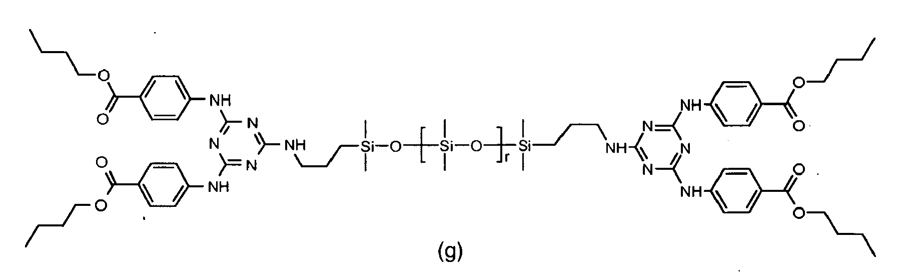

### Première étape : préparation du 2,4-bis(4'-diylamino benzoate de n-butyle)-6-chloro-s-triazine :

A une solution de chlorure de cyanuryle (54,36 g, 0,295 mole) dans 500 ml de dioxane et 50 ml d'eau, on ajoute goutte à goutte à 5°C simultanément du para-amino benzoate de n-butyle (113,94 g, 0,59 mole) et une solution de carbonate de potassium (40,68 g, 0,295 mole) dans 50 ml d'eau de telle sorte que le pH se situe entre 3 et 6,5. On maintient pendant 1 heure 30 minutes à 5°C. Un précipité se forme dans le milieu qui correspond à la s-triazine monosubstituée. On chauffe progressivement à 70°C et ajoute le deuxième équivalent de carbonate de potassium (40,68 g, 0,295 mole) dans 50 ml d'eau. L'agitation est ensuite maintenue 5 heures à 70°C. On refroidit et on filtre le mélange réactionnel. Le précipité formé est lavé à l'eau, essoré et séché. Après recristallisation dans du dioxane/eau, on obtient après séchage sous vide 52,5 g (Rendement : 36%) du premier jet de recristallisation de 2,4-bis-(4'-diylamino benzoate de n-butyle)-6-chloro-s-triazine sous forme d'une poudre blanche.

### Deuxième étape : préparation du dérivé de l'exemple 7 :

Sous barbotage d'azote, un mélange du produit précédent (2 g, 4x10-3 mole), d'aminopropyl terminated polydimethylsiloxane (DMS-A-11 de chez Gelest)(2,13 g, 2x10-3 mole) et de pyridine (0,32 ml, 4x10-3 mole) dans 40 ml de toluène est chauffé à 70°C pendant 5 heures. On refroidit, on ajoute du dichlorométhane et on lave la phase organique 3 fois à l'eau. On obtient après séchage de la phase organique et évaporation des solvants une huile marron. Après traitement au noir dans l'éthanol à chaud et filtration sur Célite, on obtient 3,3 g (Rendement : 70%) du dérivé de l'exemple 7 sous forme d'une gomme marron clair : UV (Ethanol) : λmax = 311 nm , E1% = 916.

### EXEMPLE 8 : Préparation du butyl 4-{[4-{[4-(butoxycarbonyl)phenyl]amino}-6-({3-[diethoxy(methyl)silyl]propyl}amino)-1,3,5-triazin-2-yl]amino}benzoate:

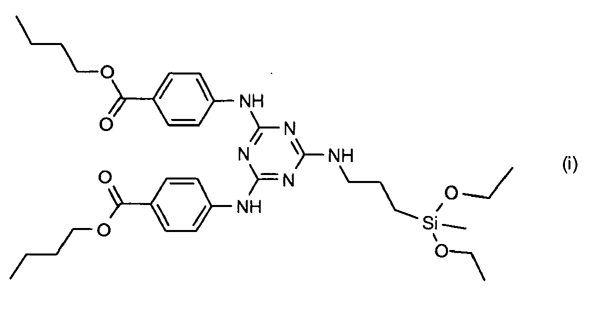

Sous barbotage d'azote, le mélange hétérogène du produit de la première étape de l'exemple 7 (20 g, 0,04 mole) et d'aminopropyl diéthoxy méthyl silane (15,37 g, 0 ,08 mole) est chauffé progressivement jusqu'à 70°C. Au bout d'une heure, on refroidit, on ajoute du dichlorométhane et on lave la phase organique 3 fois à l'eau. On obtient après séchage de la phase organique et évaporation des solvants puis par une recristallisation dans l'heptane 21 g (Rendement 80%) d'un solide blanc du dérivé de l'exemple 8 : UV (Ethanol) : λmax = 311 nm , E1% = 1197.

### EXEMPLE 9 : Préparation du dérivé statistique de formule (1a,III) obtenu par polymérisation du dérivé de l'exemple (8) avec du D5 +MM : R1 = n-butyl, X = O, n = 0, W = H, a = 1, b = 2, R = CH3, Z = CH2 :

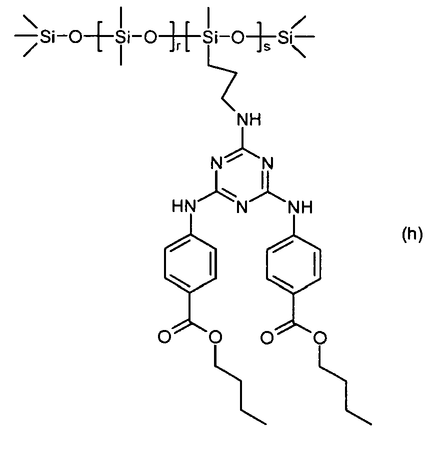

Sous barbotage d'azote, le mélange hétérogène du produit de l'exemple 8 (1 g, 1,53x10-3 mole), de decamethylcyclopentasiloxane (D5) (0,57 g, 1,53x10-3 mole), d'hexamethyldisiloxane (MM) (0,062 g, 0,38x10-3 mole) et d'acide chlorhydrique concentré (0,1 ml) est vivement agité dans un mélange de 10 ml de toluène et 1 ml d'eau. On chauffe progressivement jusque 70°C et laisse à cette température pendant 2 heures. Après refroidissement à température ambiante et dilution à l'eau, on filtre le tout. Le précipité obtenu est lavé à l'eau et séché. On obtient ainsi 0,56 g d'une poudre blanche du dérivé de l'exemple 9 :
UV (Ethanol) : λmax = 311 nm , E1% = 892.

### EXEMPLES 10a et 10b : Préparation des dérivés : butyl 4-[(4-{[4-(butoxycarbonyl)phenyl]amino}-6-{[3-(1-hydroxy-1,3,3,3-tetramethyldisiloxanyl)propyl]amino}-1,3,5-triazin-2-yl)amino]benzoate et dibutyl 4,4'-{[6-({3-[dihydroxy(methyl)silyl]propyl}amino)-1,3,5-triazine-2,4-diyl]diimino}dibenzoate obtenu par traitement acide du dérivé de l'exemple (2):

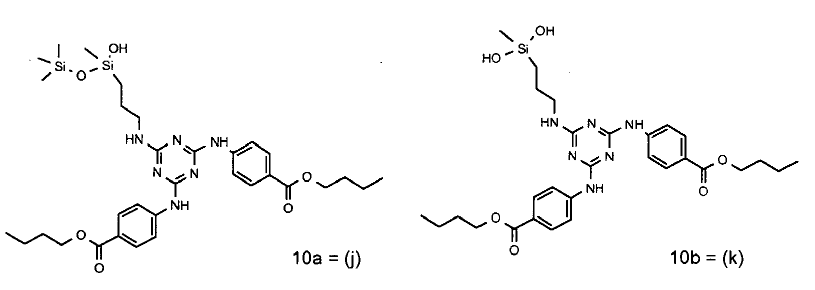

Au dérivé de l'exemple 2 (10 g, 0,013 mole) solubilisé dans 500ml du mélange ethanol/isopropanol dans le rapport 80:20, on ajoute 160ml d'acide chlorhydrique 0,1 N et 340ml du mélange ethanol/isopropanol dans le rapport 80:20. On laisse sous agitation à température du labo pendant 5 heures. Cette solution est neutralisée avec de la soude à 0,4% jusqu'à un pH de 7. On y ajoute 1 litre d'eau et la solution est lyophilisée. Les lots lyophilisés sont rassemblés pour donner 6,5g d'une poudre beige clair qui contient en pourcentages relatifs par HPLC environ 28% du dérivé de l'exemple 10a et environ 10% du dérivé de l'exemple 10b. Cette poudre a été fractionnée par chromatographie de partage centrifuge (avec systèmes biphasiques composés d'heptane, d'acétate d'éthyle, de méthanol et d'eau dans différentes proportions) pour donner 0,58g du dérivé de l'exemple 10a sous forme de poudre blanche :
UV (Ethanol) : λmax = 312 nm , E1% = 1228
et 0,42g du dérivé de l'exemple 10b sous forme d'une poudre blanche.

### EXEMPLE 11 : Préparation du mélange 7:93 du butyl 4-({4-{[4-(butoxycarbonyl)phenyl]amino}-6-[(2-{1,3,3,3-tetramethyl-1-[(trimethylsilyl)oxy]disiloxanyl}propyl)aminol-1,3,5-triazin-2-yl}amino)benzoate et du 2,4-bis(4'-diylamino benzoate d'éthyle)-6-{[1,3,3,3-tétraméthyl-1 - [(triméthylsilyl)oxy]disiloxanyl]propyl-3-ylamino}-s-triazine:

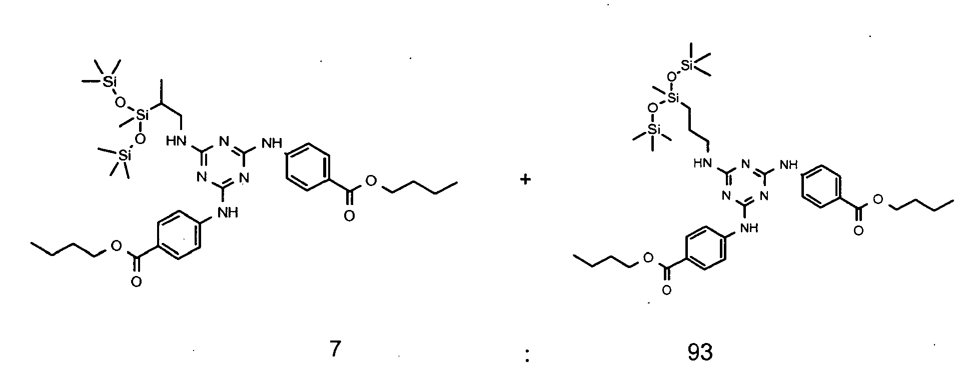

A une solution de chlorure de cyanuryle (19,8 g, 0,11 mole) et de lutidine (11,5g, 0,11 mole) dans 100 ml d'acétate d'éthyle, on ajoute goutte à goutte à 0°C en 30 minutes un mélange 7/93 de 2-{1,3,3,3-tetramethyl-1-[(trimethylsilyl)oxy]disiloxanyl}propan-1-amine et de 3-{1,3,3,3-tetramethyl-1-[(trimethylsilyl)oxy]disiloxanyl}propan-1-amine (30 g, 0,11 mole). On laisse sous agitation à 0°C pendant 30 minutes, puis à 10°C pendant 30 minutes, enfin à 20°C pendant 30 minutes. On ajoute ensuite de la pyridine (17,4g, 0,22 mole) et le para-amino benzoate de n-butyle (41,4 g, 0,22 mole) et le mélange est chauffé à 70°C pendant 3 heures. Après refroidissement du mélange réactionnel, on lave avec 2 fois 100ml d'une solution saturée de chlorure de sodium. Cette phase organique est passée sur lit de Silice et le gateau rincé avec 80ml d'acétate d'éthyle. Cette phase organique est séchée sur sulfate de sodium et les solvants évaporés. Le résidu obtenu est cristallisé dans l'Heptane. On obtient ainsi 50,2g d'un solide jaune pâle. Ce solide est recristallisé dans un mélange Heptane/EtOAc 98 :2 pour obtenir 49 g (Rendement : 60%) du mélange 7:93 des 2 isomères de l'exemple 11 sous forme d'une poudre blanche :
Pf : 165-167°C,
UV (Ethanol) : λmax = 312 nm , E1 % = 1040.

### EXEMPLE 12 : Préparation du mélange 50:50 du butyl 4-({4-{[4-(butoxycarbonyl)phenyl]amino}-6-[(2-{1,3,3,3-tetramethyl-1-[(trimethylsilyl)oxy]disiloxyanyl}propyl)amino]-1,3,5-triazin-2-yl}amino)benzoate et du 2,4-bis(4'-diylamino benzoate d'éthyle)-6-{[1,3,3,3-tétraméthyl-1-[(triméthylsilyl)oxy]disiloxanyl]propyl-3-ylamino}-s-triazine :

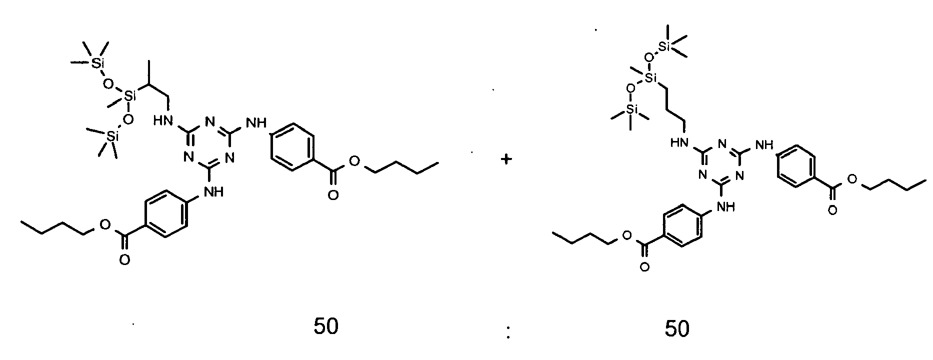

### Première étape : préparation du mélange 50 :50 du 4,6-dichloro-N-(2-{1,3,3,3-tetramethyl-1-[(trimethylsilyl)oxy]disiloxanyl}propyl)-1,3,5-triazin-2-amine et du 4,6-dichloro-N-(3-{1,3,3,3-tetramethyl-1-[(trimethylsilyl)oxy]disiloxanyl}propyl)-1,3,5-triazin-2-amine:

A une solution de chlorure de cyanuryle (32,5 g, 0,176 mole) dans 180 ml d'acétone, on ajoute goutte à goutte à 0°C un mélange 15/85 de 2-{1,3,3,3-tetramethyl-1-[(trimethylsilyl)oxy]disiloxanyl}propan-1-amine et de 3-{1,3,3,3-tetramethyl-1-[(trimethylsilyl)oxy]disiloxanyl}propan-1-amine (49,3 g, 0,176 mole) et une solution de bicarbonate de sodium (14,8 g, 0,176 mole) dans 210 ml d'eau de telle sorte que le pH se situe entre 4 et 5,8. En fin d'introduction, le pH est de 5,3. L'agitation est ensuite maintenue 1 heure 30 minutes à 10°C, puis laissé à température du labo. Le précipité formé est filtré, lavé à l'eau, essoré et séché. On obtient 72,4 g (Rendement : 96%) des dérivés isomères attendus dans le rapport 15/85 sous forme d'une poudre blanche (Pf : 59°C).

Un fractionnement de 20 g du mélange précédent sur 2 colonnes chromatographiques de Silice successives (éluant Heptane/EtOAc 95:5) a été réalisé. On a pu obtenir 3,54g du mélange isomérique 50:50 et utilisé tel quel dans l'étape suivante.

### Deuxième étape : préparation du dérivé de l'exemple 12 :

Le mélange du produit précédent (3 g, 0,007 mole), de para-amino benzoate de n-butyle (2,7 g, 0,014 mole) et de bicarbonate de sodium (1,18g, 0,014 mole) en suspension dans 30 ml de toluène est chauffé à 70°C pendant 5 heures. On refroidit et ajoute du dichlorométhane. Après 2 lavages à l'eau, la phase organique est séchée sur sulfate de sodium et les solvants évaporés. Le résidu obtenu est cristallisé dans un mélange Heptane/EtOAc 50:5. Le précipité est ensuite purifié par colonne chromatographique de Silice (éluant : CH2Cl2/EtOAc 95:5) pour obtenir 3,7 g (Rendement : 71%) du mélange 50:50 des 2 isomères de l'exemple 12 sous forme d'une poudre blanche :
Pf : 165-167°C,
UV (Ethanol) : λmax = 312 nm , E1% = 1149.

### EXEMPLE 13 : Préparation du dérivé de formule (Ia,III) : R₁ = n-butyl, X = O, n = 0, B = CH₃, W=H, Z = CH₂, R₇ = CH₃, s = 1, r=0:

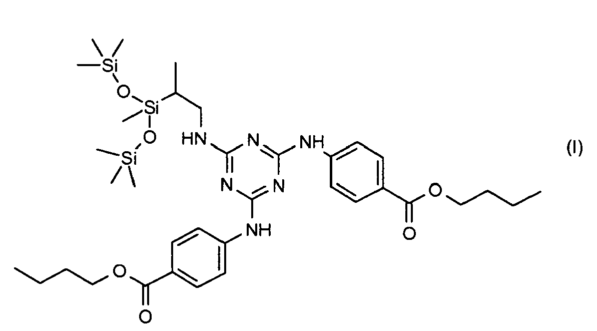

### Première étape : préparation du 4,6-dichloro-N-(2-{1,3,3,3-tetramethyl-1-[(trimethylsilyl)oxy]disiloxanyl}propyl)-1,3,5-triazin-2-amine:

Le mélange isomérique 15:85 de la première étape de la synthèse de l'exemple 12 a été fractionné par chromatographie de partage centrifuge (système biphasique : Heptane/Acétonitrile/Eau 50:49:1) pour donner 2,0 g de 4,6-dichloro-N-(2-{1,3,3,3-tetramethyl-1-[(trimethylsilyl)oxy]disiloxanyl}propyl)-1,3,5-triazin-2-amine engagé tel quel dans l'étape suivante :

### Deuxième étape : préparation du dérivé de l'exemple 13 :

Le produit précédent (2 g, 0,0047 mole) est solubilisé dans 18ml de toluène. On y ajoute de la pyridine (0,8ml, 0,009 mole) et du para-amino benzoate de n-butyle (1,8 g, 0,009 mole). On chauffe à 70°C sous agitation pendant 3 heures. On refroidit et verse la solution sur un lit de Silice et rince le gateau avec 80ml de toluène. Après évaporation du solvant, le solide beige marron obtenu est cristallisé dans 30ml d'heptane. On obtient ainsi 2,2g (Rendement 63%) du dérivé de l'exemple 13 sous forme d'une poudre beige clair :
Pf: 149-151°C,
UV (Ethanol) : λmax = 312 nm , E1% = 955.

### EXEMPLE 14 : Préparation du dérivé de formule (Ia,III) : R₁ = n-butyl, X = O, n = 0, B = CH₃, W = H, Z = CH₂, R₇ = CH₃, s = 2, r = 0:

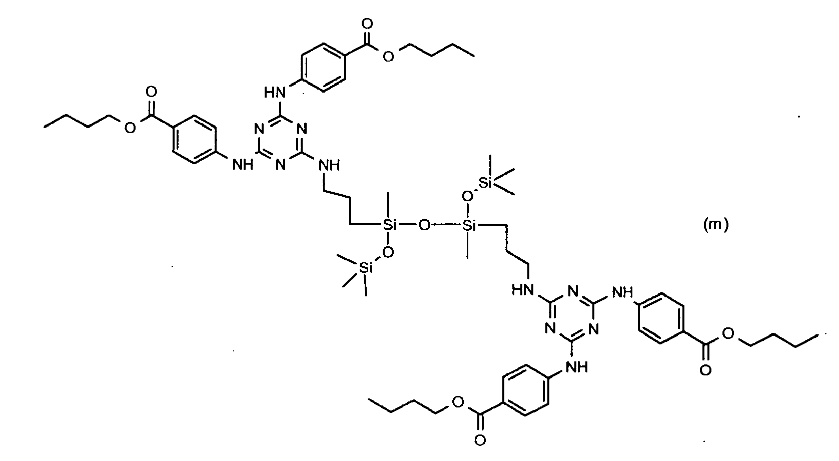

Au dérivé de l'exemple 2 (1 g, 0,0013 mole) solubilisé dans 50ml du mélange ethanol/isopropanol dans le rapport 80:20, on ajoute 25ml d'acide chlorhydrique 1 N. On laisse sous agitation à température du labo pendant 4 heures. Cette solution est neutralisée avec de la soude à 35% jusqu'à un pH de 7. Les solvants sont évaporés sous vide. On obtient 0,8g d'une poudre beige clair qui contient en pourcentage relatif par HPLC environ 37% du dérivé de l'exemple 14. Cette poudre a été fractionnée par chromatographie de partage centrifuge (avec système biphasique composés d'heptane, d'acétate d'éthyle, de méthanol et d'eau) pour donner 0,18g du dérivé de l'exemple 14 sous forme de poudre blanche :
UV (Ethanol) : λmax = 312 nm , E1% = 1109.

### EXEMPLES DE FORMULATION 15 à 17

| **Nom chimique** | **Ex 15** | **Ex16** | **Ex 17** |
|---|---|---|---|
| Phase A | | | |
| Composé s-triazine de l'exemple 13 | 3 | | |
| Composé s-triazine de l'exemple 2 | | 3 | |
| Composé s-triazine de l'exemple 3 | | | 5 |
| Butylmethoxydibenzoylmethane (PARSOL 1789) | 2 | 2 | 2 |
| 2,4,6-tris[p-(2'-éthylhexyl-1'-oxycarbonyl)anilino]-1,3,5-triazine (UVINUL T150) | 3 | 2 | 3 |
| C12-15 alkyl benzoate | 15 | 15 | 15 |
| Alcool cétylique | 0,5 | 0,5 | 0,5 |
| Acide stéarique | 1,5 | 1,5 | 1,5 |
| Mélange mono-stéarate de glycéryle / stéarate de PEG (100 OE) | 1 | 1 | 1 |
| Mélange de cétylstéaryl glucoside et d'alcools cétylique, stéarylique | 2 | 2 | 2 |
| Dimethicone | 0,50 | 0,50 | 0,50 |
| Triethanolamine | 0,45 | 0,45 | 0,45 |
| Conservateur | 1 | 1 | 1 |
| Dioxyde de titane | | 5 | |

| Phase B | | | |
|---|---|---|---|
| Glycérol | 5 | 5 | 5 |
| 5Complexant | 0,1 | 0,1 | 0,1 |
| Phosphate de mono cétyle | 1 | 1 | 1 |
| Eau | qsp 100G | qsp 100G | qsp 100G |

| Phase C | | | |
|---|---|---|---|
| Gomme de Xanthane | 0,2 | 0,2 | 0,2 |
| Copolymère acide acrylique/méthacrylate de stéaryle | 0,2 | 0,2 | 0,2 |
| Iso-Hexadecane 1 | 1 | 1 | 1 |
| Cyclopentasiloxane | | 1 | |

| Phase D | | | |
|---|---|---|---|
| Triethanolamine | qsp pH | qsp pH | qsp pH |

### Mode opératoire

On chauffe la phase grasse (A) à 70°C. On chauffe la phase aqueuse (B) dans le bécher final. On prépare la phase (C) : dispersion des poudres dans l'huile. Sous agitation Rotor Stator, on émulsionne la phase grasse dans la phase aqueuse. On introduit la phase (C) sous agitation plus rapide puis on laisse sous agitation lente jusqu'au retour à température ambiante. On neutralise (D) puis on conditionne.

## Revendications

1. Composition comprenant au moins, dans un support physiologiquement acceptable, un système filtrant, **caractérisée par le fait qu'**elle comprend :
(a) au moins un filtre UV du type dérivé du dibenzoylméthane et
(b) au moins un filtre UV du type 1,3,5-triazine photosensible en présence d'un dérivé du dibenzoylméthane et
(c) au moins un composé s-triazine siliciée substituée par deux groupements aminobenzoates ou aminobenzamides de formule (I) suivante ou l'une de ses formes tautomères : 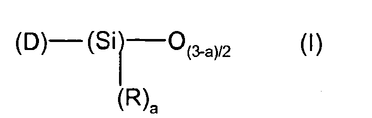 dans laquelle
- R, identiques ou différents représentent un radical alkyle en C₁-C₃₀, linéaire ou ramifié et éventuellement halogéné ou insaturé, un radical aryle en C₆-C₁₂, un radical alkoxy en C₁-C₁₀ ou le groupe triméthylsilyloxy ;
- a = 0 à 3;
- le groupe D désigne un composé s-triazine de formule (II) suivante : 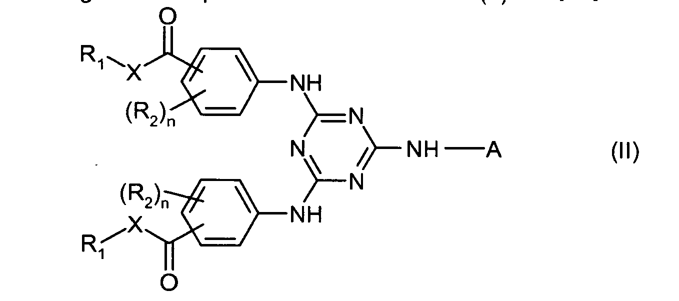
où
- X représente -O- ou -NR₃-, avec R₃ qui représente l'hydrogène ou un radical alkyle en C₁-C₅,
- R₁ représente un radical alkyle en C₁-C₂₀, linéaire ou ramifié et éventuellement insaturé et pouvant contenir un atome de silicium, un groupe cycloalkyle en C₅-C₂₀, éventuellement substitué par 1 à 3 radicaux alkyles en C₁-C₄, linéaires ou ramifiés, le groupe -(CH₂CHR₄-O)ₘR₅ ou le groupe -CH₂-CH(OH)-CH₂-O-R₆,
- R₄ représente l'hydrogène ou méthyle ; le groupement (C=O)XR1 pouvant être en position ortho, méta ou para du groupement amino,
- R₅ représente l'hydrogène ou un groupe alkyle en C1-C8,
- R₆ représentent l'hydrogène ou un groupe alkyle en C4-C8,
- m est un nombre entier allant de 2 à 20,
- n = 0 à 2,
- R₂, identiques ou différents, représentent un radical hydroxy, un radical alkyle en C₁-C₈, linéaire ou ramifié, un radical alcoxy en C1-C8, deux R2 adjacents d'un même noyau aromatique pouvant former ensemble un groupement alkylidène dioxy dans lequel le groupe alkylidène contient 1 ou 2 atomes de carbone,
- A est un radical divalent choisi parmi méthylène, -[CH(Si(CH₃)₃]-, éthylène ou un groupe répondant à l'une des formules (III), (IV) ou (V) suivantes : 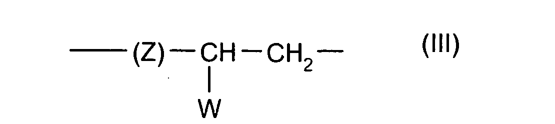 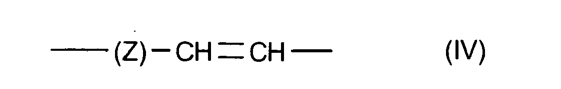 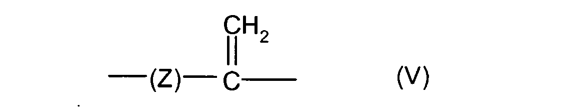
dans lesquelles :
- Z est un diradical alkylène en C₁-C₁₀, linéaire ou ramifié, saturé ou insaturé, éventuellement substitué par un radical hydroxyle ou des oxygènes et pouvant éventuellement contenir un groupement amino,
- W représente un atome d'hydrogène, un radical hydroxyle ou un radical alkyle en C₁-C₈, linéaire ou ramifié, saturé ou insaturé,

2. Composition selon la revendication 1, où le composé de formule (I) est sous la forme tautomère de formule (I') suivante : 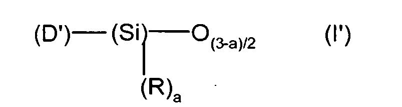 dans laquelle le groupe D' désigne un composé s-triazine de formule (II') suivante :

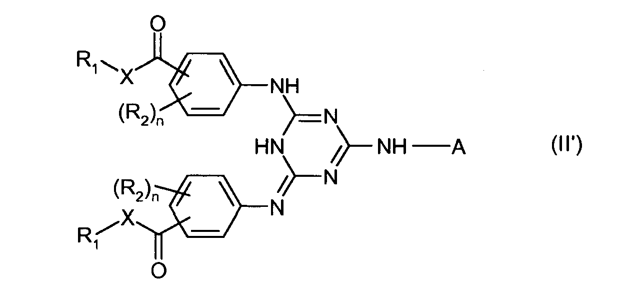

3. Composition selon la revendication 1 ou 2, où le composé de formule (I) comprend en plus des unités de formule (R)_{b}-(SI)(O)_{(4-b)/2} dans lesquelles :
R a la même signification que dans la formule (I),
b = 1, 2 ou 3.

4. Composition selon la revendication 3, où les composés de formule (I) sont choisis parmi ceux pour lesquels au moins l'une, et encore plus préférentiellement l'ensemble, des caractéristiques suivantes sont remplies :
R est méthyle,
a = 1 ou 2,
X est O,
R₁ est un radical en C₄-C₅
n=0,
le groupement (C=O)XR₁ est en position para vis-à-vis du groupement amino, Z=-CH₂-,
W = H.

5. Composition selon l'une quelconque des revendications 1 à 4, où les composés de formule (I) sont choisis parmi ceuxx répondant à l'une des formules (Ia), (Ib) ou (Ic) suivantes : 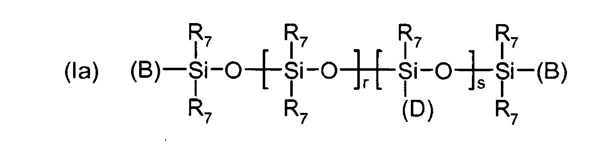 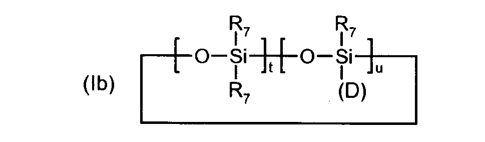
(Ic) (D)-Si(R8)3
dans lesquelles :
- (D) répond à la formule (II) telle que définie dans les revendications précédentes,
- R₇, identiques ou différents, sont choisis parmi les radicaux alkyles linéaires ou ramifiés en C₁-C₂₀, phényle, 3,3,3-trifluoropropyle et triméthylsilyloxy ou le radical hydroxy,
- R₈, identiques ou différents, sont choisis parmi les radicaux alkyles et alcényles, linéaires ou ramifiés en C₁-C₂₀ , les radicaux hydroxy ou phényle,
- (B), identiques ou différents sont choisis parmi les radicaux R₇ et le radical (D),
r est un nombre entier compris entre 0 et 200 inclusivement,
- s est un nombre entier allant de 0 à 50 et si s = 0, au moins l'un des deux symboles (B) désigne (D),
- u est un nombre entier allant de 1 à 10,
- t est un nombre entier allant de 0 à 10, étant entendu que t + u est égal ou supérieur à 3 ainsi que leurs formes tautomères.

6. Composition selon la revendication 5, où les composés de formule (la) ou (Ib) sont des oligomères ou polymères statistiques présentant au moins l'une, et encore plus préférentiellement l'ensemble, des caractéristiques suivantes :
- R₇ est le radical méthyle ou le radical hydroxy
- B est préférentiellement méthyle.

7. Composition selon la revendication 6, où le composé de formule (I) est choisi parmi les composés de formules (a) à (m) suivantes ainsi que leurs formes tautomères :

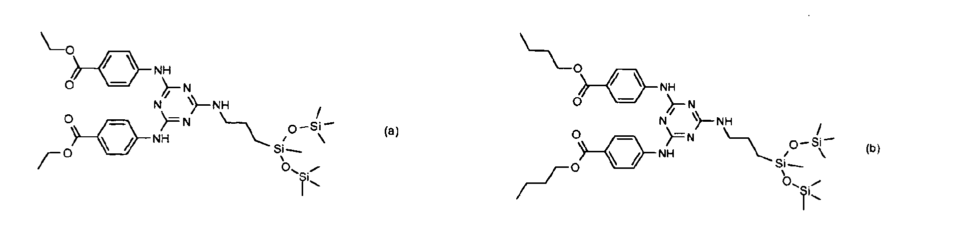

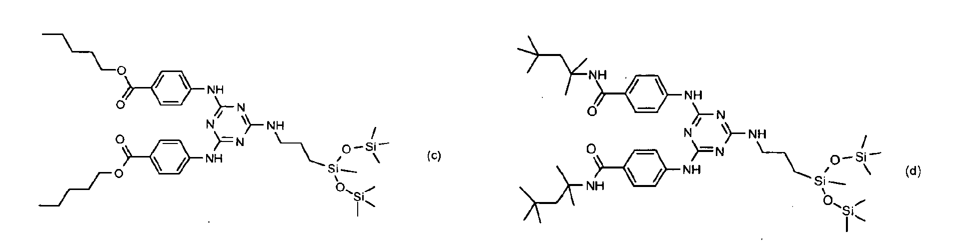

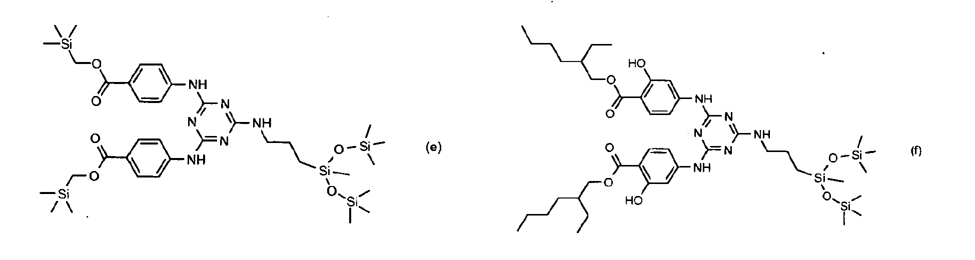

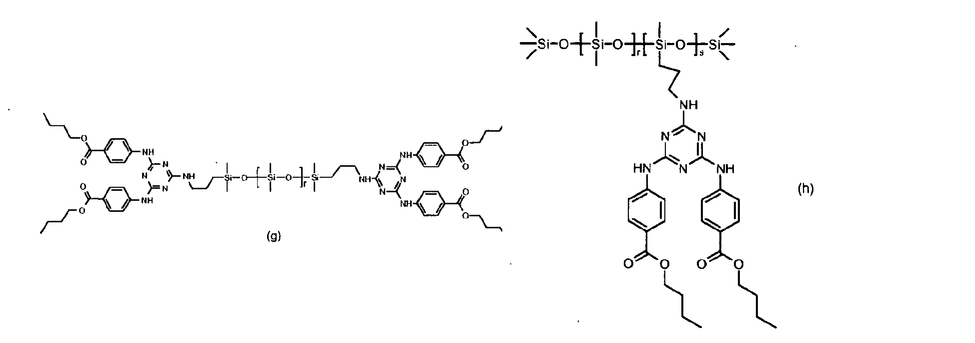

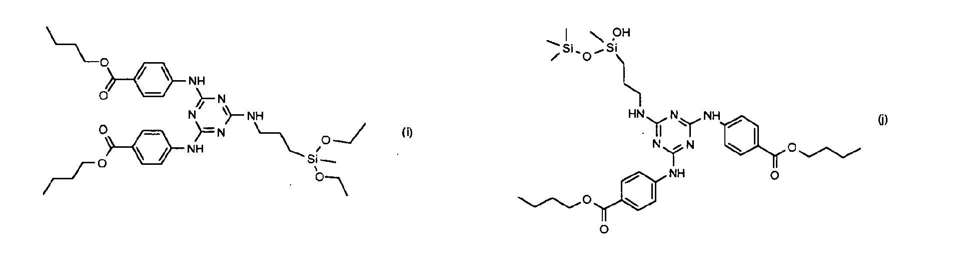

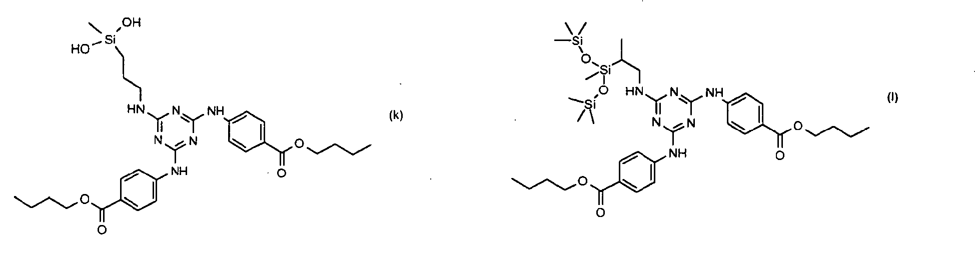

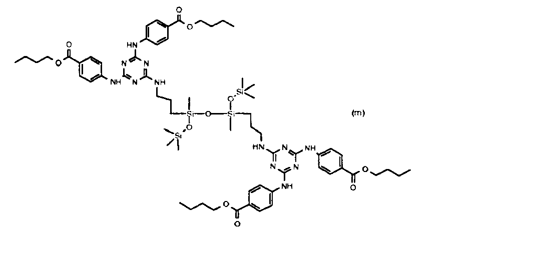

8. Composition selon la revendication 7, où le composé de formule (I) est le composé 2,4-bis(4'-diylamino benzoate de n-butyle)-6-{[1,3,3,3-tétraméthyl-1-[(triméthylsilyl)oxy]disiloxanyl]propyl-3-ylamino}-s-triazine de structure (b) :

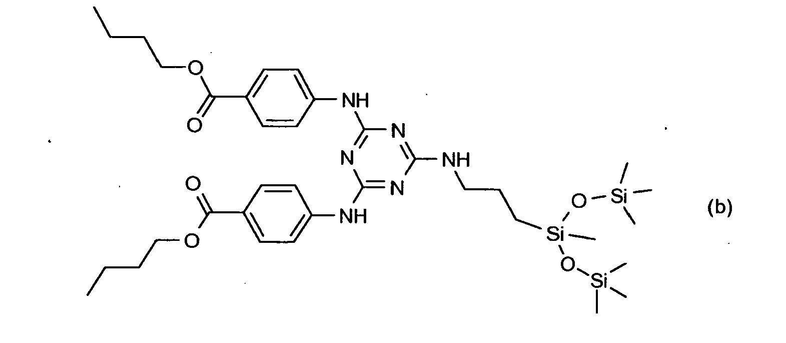

9. Composition selon l'une quelconque des revendications précédentes, où le ou les composés s-triazine de formule (I) sont présents à des teneurs allant de 0,01 à 20% en poids et plus préférentiellement de 0,1 à 10 % et encore plus préférentiellement de 0,1 à 6% en poids par rapport au poids total de la composition.

10. Composition selon l'une quelconque des revendications 1 à 9, **caractérisée par le fait que** le dérivé de dibenzoylméthane est choisi parmi :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

11. Composition selon la revendication 10, **caractérisée par le fait que** le dérivé de dibenzoylméthane est le 4-(ter.-butyl) 4'-méthoxy dibenzoylméthane.

12. Composition selon l'une quelconque des revendications 1 à 11, où le dérivé du dibenzoylméthane est présent à des teneurs allant de 0,01 à 20% en poids et plus préférentiellement de 0,1 % à 10 % en poids, et encore plus particulièrement de 0,1 à 6% en poids par rapport au poids total de la composition.

13. Composition selon l'une quelconque des revendications précédentes, où le ou les filtres UV du type 1,3,5-triazine lipophiles non-siliciés sont choisis parmi les dérivés de 1,3,5-triazine de formule (VIII) suivante : 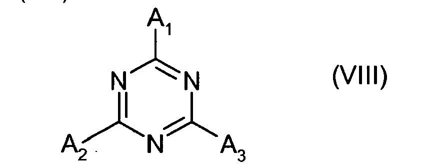 dans laquelle les radicaux A₁, A₂ et A₃, identiques ou différents sont choisis parmi les groupes de formules (IX) : 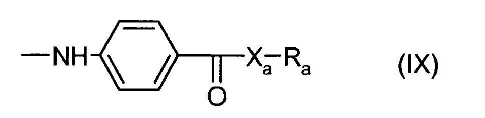 dans laquelle :
- Xₐ, identiques ou différents, représentent l'oxygène ou le radical -NH-;
- Rₐ, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (X), (XI) ou (XII) suivantes : 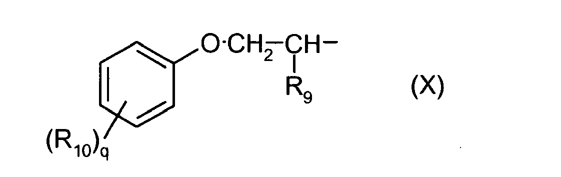 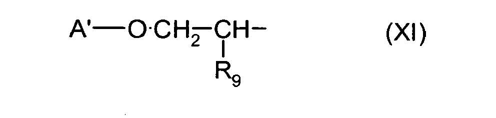 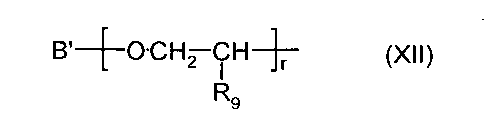
dans lesquelles :
- R₉ est l'hydrogène ou un radical méthyle;
- R₁₀ est un radical alkyle en C₁-C₉;
- q est un nombre entier allant de 0 à 3;
- r est un nombre entier allant de 1 à 10;
- A' est un radical alkyle en C₄-C₈ ou un radical cycloalkyle en C₅-C₈;
- B' est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄.

14. Composition selon la revendication 13, où les dérivés de 1,3,5-triazine répondant à la formule (VIII) sont choisis parmi ceux pour lesquels les A₁, A₂ et A₃ sont de formule (IX) et présentent les caractéristiques suivantes :
- un des radicaux Xₐ-Rₐ représente le radical -NH-Rₐ avec Rₐ choisi parmi : un radical cycloalkyle en C₅-C₁₂ éventuellement substitué par un ou plusieurs radicaux alkyles en C₁-C₄; un radical de formule (X), (XI) ou (XII) ci-dessus dans lesquelles B' est un radical alkyle en C₁-C₄ et R₁₀ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (X), (XI) ou (XII) ci-dessus dans lesquelles B' est un radical alkyle en C₁-C₄ et R₁₀ est le radical méthyle.

15. Composition selon la revendication 13, où les dérivés de 1,3,5-triazine répondant à la formule (VIII) sont choisis parmi ceux pour lesquels les A₁, A₂ et A₃ sont de formule (IX) et présentent les caractéristiques suivantes :
- un ou deux Xₐ-Rₐ représente le radical -NH-Rₐ, avec Rₐ choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (X), (XI) ou (XII) ci-dessus dans lesquelles B' est un radical alkyle en C₁-C₄ et R₁₀ est le radical méthyle ;
- le ou les deux autres Xₐ-Rₐ étant le radical -O-Rₐ avec Rₐ, identiques ou différent choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (X), (XI) ou (XII) ci-dessus dans lesquelles : B' est un radical alkyle en C₁-C₄ et R₁₀ est le radical méthyle.

16. Composition selon la revendication 15, où le dérivé de 1,3,5-triazine de formule (VIII) est la 2-[(p-(tertiobutylamido)anilino]-4,6-bis-[(p-(2'-éthylhexyl-1'-oxycarbonyl)anilino]-1,3,5-triazine répondant à la formule suivante : 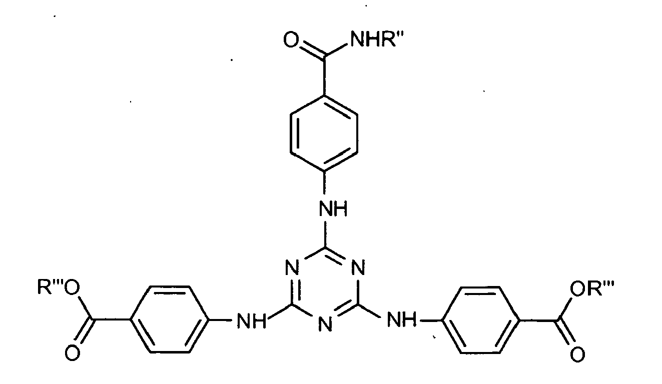 dans laquelle R"' désigne un radical éthyl-2 hexyle et R" désigne un radical tert-butyle.

17. Composition selon la revendication 16, où les dérivés de 1,3,5-triazine répondant à la formule (VIII) sont choisis parmi ceux pour lesquels les A₁, A₂ et A₃ sont de formule (IX) et présentent les caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

18. Composition selon la revendication 14, où le dérivé de 1,3,5-triazine de formule (VIII) est la 2,4,6-tris[p-(2'-éthylhexyl-1'-oxycarbonyl)anilino]-1,3,5-triazine et répondant à la formule suivante : 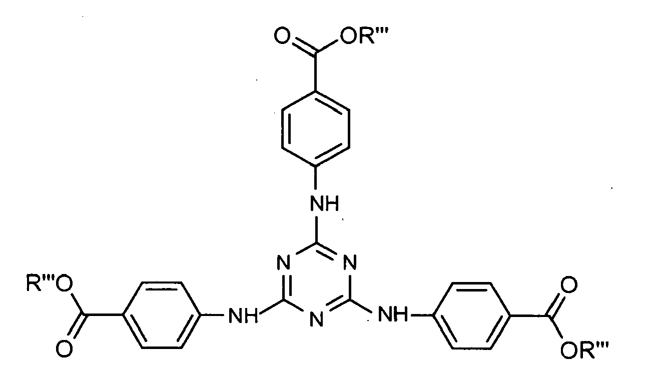 dans laquelle R"' désigne un radical 2-éthyl hexyle.

19. Composition selon l'une quelconque des revendications 1 à 18, où le ou les composés 1,3,5 triazines photosensibles sont présents à des teneurs allant de 0,01 à 20% en poids et plus préférentiellement de 0,1 % à 10 % en poids, et encore plus particulièrement de 0,1 à 6% en poids par rapport au poids total de la composition.

20. Composition selon l'une quelconque des revendications précédentes, **caractérisée par le fait qu'**elle contient en plus d'autres agents photoprotecteurs organiques ou inorganiques actifs dans l'UV-A et/ou l'UV-B.

21. Composition selon la revendication 11, où les agents photoprotecteurs organiques complémentaires sont choisis parmi es anthranilates ; les dérivés cinnamiques ; les dérivés salicyliques, les dérivés du camphre ; les dérivés de la benzophénone ; les dérivés de β, β -diphénylacrylate ; les dérivés de benzalmalonate ; les dérivés de benzimidazole ; les imidazolines ; les dérivés bis-benzoazolyle ; les dérivés de l'acide p-aminobenzoïque (PABA) ; les dérivés de benzoxazole ; les polymères filtres et silicones filtres ; les dimères dérivés d'α-alkylstyrène ; les 4,4-diarylbutadiènes et leurs mélanges.

22. Composition selon la revendication 19, **caractérisée par le fait que** le ou les filtres UV organiques sont choisis parmi les composés suivants :
Ethylhexyl Methoxycinnamate
Homosalate
Ethylhexyl Salicylate,
Octocrylene,
Phenylbenzimidazole Sulfonic Acid,
Disodium Phenyl Dibenzimidazole Tetra-sulfonate,
Terephthalylidene Dicamphor Sulfonic Acid
Benzophenone-3,
Benzophenone-4,
Benzophenone-5,
4-Methylbenzylidene camphor,
Polysilicone-15
1,1-dicarboxy (2,2'-diméthyl-propyl)-4,4-diphénylbutadiène
et leurs mélanges.

23. Composition selon la revendication 20, **caractérisée par le fait que** les agents photoprotecteurs inorganiques complémentaires sont des pigments d'oxydes métalliques, traités ou non.

24. Composition selon la revendication 23, **caractérisée par le fait que** lesdits pigments sont choisis parmi les oxydes de titane, de zinc, de fer, de zirconium, de cérium et leurs mélanges, traités ou non.

25. Composition selon l'une quelconque des revendications 1 à 24, **caractérisée par le fait qu'**elle comprend en outre au moins un agent de bronzage et/ou de brunissage artificiel de la peau.

26. Composition selon l'une quelconque des revendications 1 à 25, **caractérisée par le fait qu'**elle comprend en outre au moins un adjuvant choisi parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants

27. Composition selon l'une quelconque des revendications 1 à 26, **caractérisée par le fait qu'**elle se présente sous la forme d'une émulsion huile-dans-eau ou eau-dans huile.

28. Utilisation d'une composition telle que définie dans l'une quelconque des revendications 1 à 27 pour la fabrication de produits pour le traitement cosmétique de la peau, des lèvres, des ongles, des cheveux, des cils, sourcils et/ou du cuir chevelu

29. Utilisation d'une composition telle que définie dans l'une quelconque des revendications 1 à 27 pour la fabrication de produits de soin de la peau, des lèvres, des ongles, des cheveux et/ou du cuir chevelu.

30. Utilisation d'une composition telles que définie dans l'une quelconque des revendications 1 à 27 pour la fabrication de produits de maquillage.

31. Procédé pour améliorer la stabilité au rayonnement UV d'un dérivé de 1,3,5-triazine photosensible en présence d'un dérivé de dibenzoylméthane tels que définis dans les revendications précédentes, **caractérisé par le fait qu'**il consiste à ajouter à ladite association au moins un composé s-triazine siliciée substituée par deux groupements aminobenzoates ou aminoberizamides de formule (I) ou l'une de ses formes tautomères tel que défini dans les revendications précédentes.

32. Utilisation d'au moins un composé s-triazine siliciée substituée par deux groupements aminobenzoates ou aminobenzamides de formule (I) ou l'une de ses formes tautomères tel que défini dans les revendications précédentes dans une composition contenant au moins un dérivé du dibenzoylméthane et au moins un dérivé de 1,3,5-triazine photosensible en présence de celui-ci tels que définis dans les revendications précédentes, dans le but d'améliorer la photostabilité dudit dérivé de 1,3,5-triazine.

## Claims

1. Composition comprising, in a physiologically acceptable support, at least one screening system, **characterized in that** it comprises:
(a) at least one UV-screening agent of the dibenzoylmethane derivative type, and
(b) at least one UV-screening agent of the 1, 3, 5-triazine type that is photosensitize in the presence of a dibenzoylmethane derivatives, and
(c) at least one siliceous s-triazine compound substituted with two aminobenzoate or aminobenzamide groups of formula (I) below, or a tautomeric form thereof: 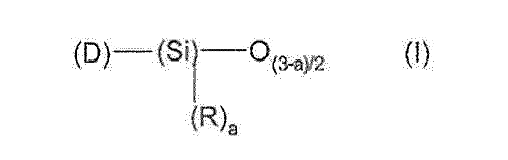 in which:
- R, which may be identical or different, represent a linear or branched and optionally halogenated or unsaturated C₁-C₃₀ alkyl radical, a C₆-C₁₂ aryl radical, a C₁-C₁₀ alkoxy radical or a trimethylsilyloxy group;
- a = 0 to 3;
- the group D denotes an s-triazine compound of formula (II) below: 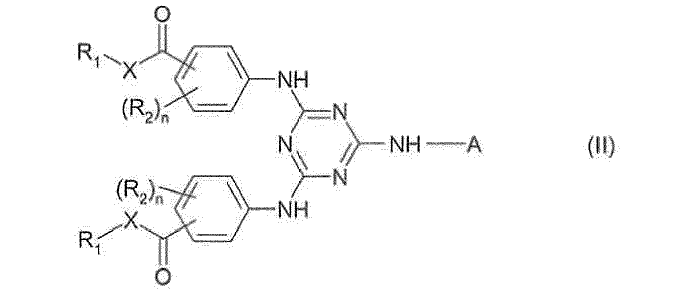
in which:
- X represents -O- or -NR₃-, with R₃ representing hydrogen or a C₁-C₅ alkyl radical,
- R₁ represents a linear or branched and optionally unsaturated C₁-C₂₀ alkyl radical possible containing a silicon atom, a C₅-C₂₀ cycloalkyl group optionally substituted, with 1 to 3 linear or branched C₁-C₄ alkyl radicals, the group -(CH₂CHR₄-O)ₘR₅ or the group -CH₂-CH(OH)-CH₂-O-R₆,
- R₄ represents hydrogen or methyl; the group (C=O)XR₁, which may be in an ortho, meta or para position relative to the amino groups
- R₅ represents hydrogen or a C₁-C₈ alkyl groups,
- R₆ represents hydrogen or a C₄-C₈ alkyl group,
- m is an integer ranging from 2 to 20,
- n = 0 to 2,
- R₂, which may be identical or different, represent a hydroxyl radicals, a linear or branched C₁-C₈ alkyl radical or a C₁-C₈ alkoxy radical, two adjacent groups R₂ on the same aromatic nucleus together possible forming an alkylidenedioxy group in which the alkylidene group contains 1 or 2 carbon atoms,
- A is a bivalent radical chosen from methylene,
- [CHSi(CH₃)₃]-, methylene and a group corresponding to one of the formulae (III), (IV) and (V) below: 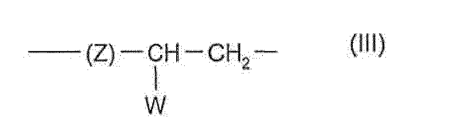 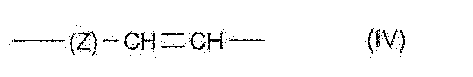 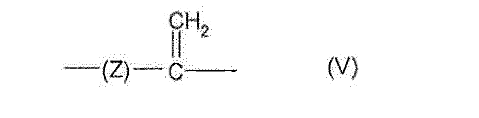
in which:
- Z is a linear or branched, saturated or unsaturated C₁-C₁₀ alkylene diradical optionally substituted with a hydroxyl radical or oxygens and possibly containing an amino group,
- W represents a hydrogen atome, a hydroxyl radical or a linear or branched, saturated or unsaturated C₁-C₈ alkyl radical.

2. Composition according to Claim 1, in which the compound of formula (1) is in the tautomeric form of formula (I') below: 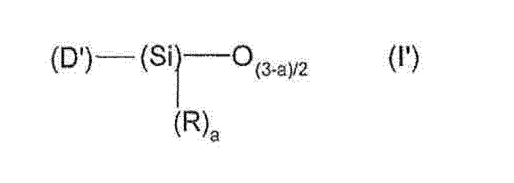 in which the group D' denotes an s-triazine compounds of formula (II') below:

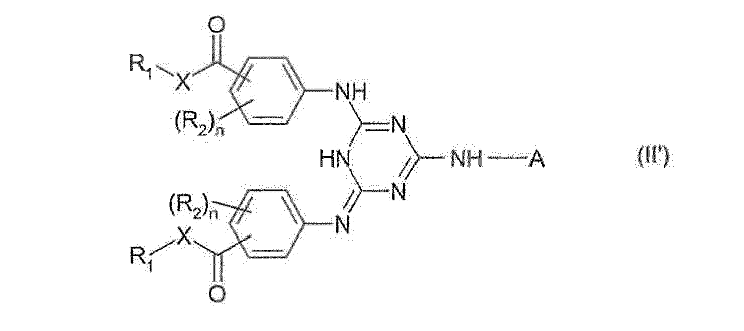

3. Composition according to Claim 1 or 2, in which the compound of formula (I) also comprises units of formula (R)_{b}-(Si)(O)_{(4-b)/2} in which:
R the same meaning as in formula (I),
b = 1, 2 or 3.

4. Composition according to Claim 3, in which the compounds of formula (I) are chosen from those for which at least one, and even more preferentially all, of the following characteristics satisfied:
R is methyl,
a = 1 or 2,
X is O,
R₁ is a C₄-C₅ radical,
n = 0,
the group (C=O)XR₁ is in the para position relative to the amino group,
Z = -CH₂-,
W = H.

5. Composition according to any one of Claims 1 to 4, in which the compounds of formula (I) are chosen from those corresponding to one of the formulae (Ia), (Ib) and (Ic) below: 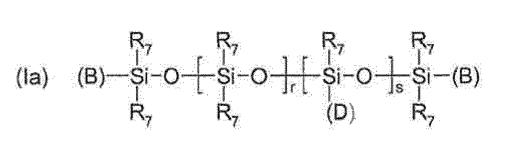 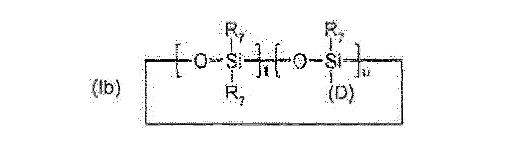
(Ic) (D)-Si(R8)3
in which:
- (D) corresponds to formula (II) as defined in the preceding claims,
- R₇, which may be identical or different, are chosen from linear or branched C₁-C₂₀ alkyl, phenyl, 3,3,3-trifluoropropyl and trimethylsilyloxy radicals or a hydroxyl radical,
- R₈, which may be identical or different, are chosen from linear or branched C₁-C₂₀ alkyl and alkenyl radicals, and hydroxyl or phenyl radicals,
- (B), which may be identical or different, are chosen from the radicals R₇ and the radical (D),
- r is an integer between 0 and 200 inclusive,
- s is an integer ranging from 0 to 50 and if s = 0, at least one of the two symbols (B) denotes (D),
- u is an integer ranging from 1 to 10,
- t is an integer ranging from 0 to 10, it being understood that t + u is greater than or equal to 3, and also the tautomeric forms thereof.

6. Composition according to Claim 5, in which the compounds of formula (Ia) or (Ib) are random oligomers or polymers having at least one, and even more preferentially all, of the following characteristics:
- R₇ is a methyl radical or a hydroxyl radical,
- B is preferentially methyl.

7. Composition according to Claim 6, in which the compound of formula (I) is chosen from the compounds of formulae (a) to (m) below, and also the tautomeric forms thereof:

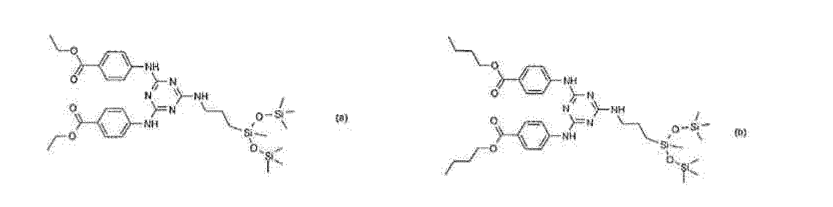

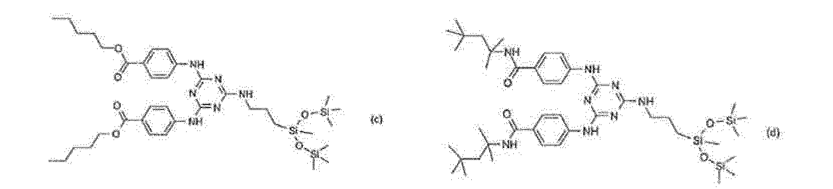

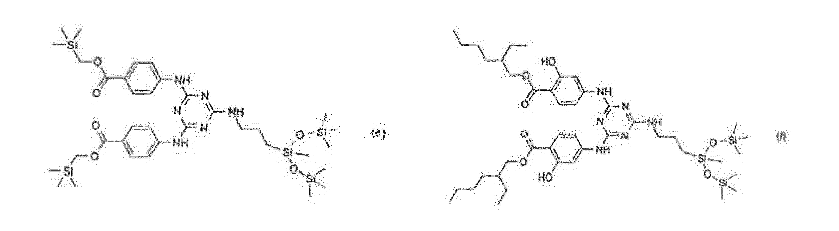

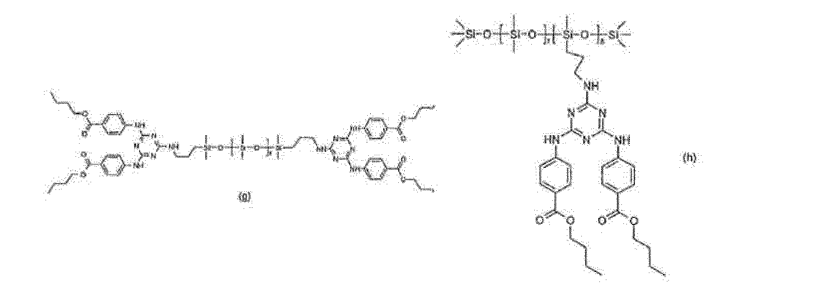

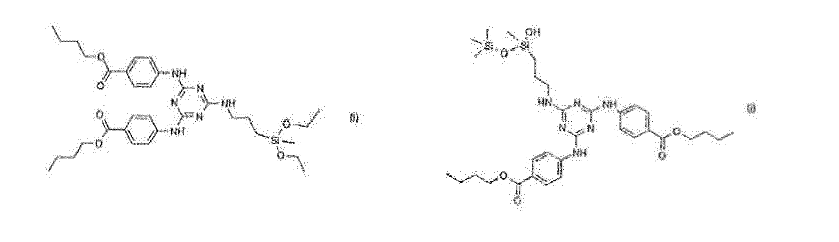

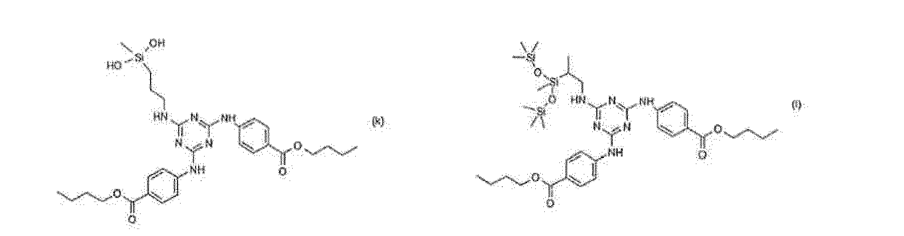

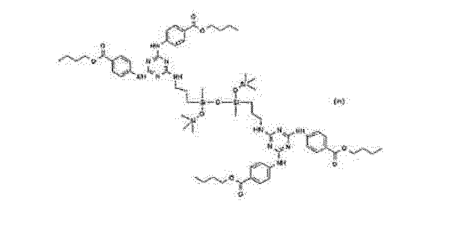

8. Composition according to Claim 7, in which the compound of formula (I) is the compound 2,4-bis(n-butyl 4'-diylaminobenzoate)-6-{[1,3,3,3-tetramethyl-1-[(trimethylsilyl)oxy]disiloxanyl]propyl-3-ylamino)-s-triazine of structure (b) :

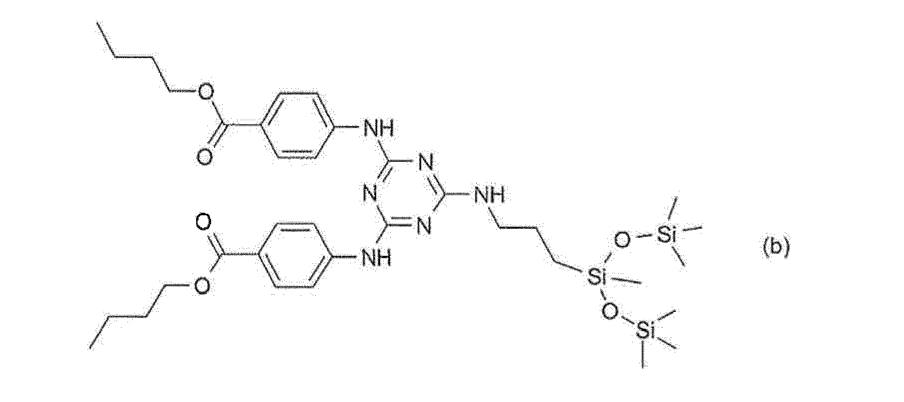

9. Composition according to any one of the preceding claims, in which: the s-triazine compound(s) of formula (I) is (are) prevent in contents ranging from 0.01% to 20% by weight, more preferentially from 0.1% to 10% and even more preferentially from 0.1% to 6% by weight relative to the total weight of the composition.

10. Composition according to any one of Claims 1 to 9, **characterized in that** the dibenzoylmethane derivative is chosen from:
- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2,5-dimethyldibenzoylmethane,
- 4,4'-diisopropyldibenzoylmethane,
- 4,4'-dimethoxydibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoylmethane,
- 2,6-dimethyl-4-tert-butyl-4'-methoxydibenzoylmethane.

11. Composition according to Claim 10, **characterized in that** the dibenzoylmethane derivative is 4-(tert-butyl)-4'-methoxydibenzoylmethane.

12. Composition according to any one of Claims 1 to 11, in which the dibenzoylmethane derivative is present in contents ranging from 0.01% to 20% by weight, more preferentially from 0.1% to 10% by weight and even more particularly from 0.1% to 6% by weight relative to the total weight of the composition.

13. Composition according to any one of the preceding claims, in which the UV-screening agent(s) of the non-siliceous lipophilic 1,3,5-triazine type are chosen from the 1,3,5-triazine derivatives of formula (VIII) below: 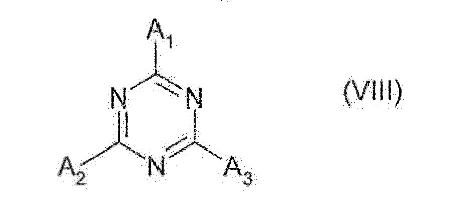 in which the radicals A₁, A₂ and A₃, which may be identical or different, are chosen from the groups of formula (IX): 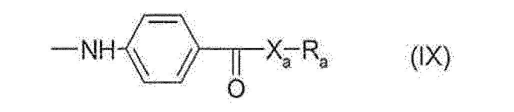 in which:
- Xₐ, which: may be identical or different, represents oxygen or the radical -NH-;
- Rₐ, which may be identical or different, are chosen from: hydrogen; an alkali metal; an ammonium radical, optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a polyoxyethylene radical comprising from 1 to 6 ethylene oxide units, the terminal OH group of which is methylated; a radical of formula (X), (XI) or (XII) below: 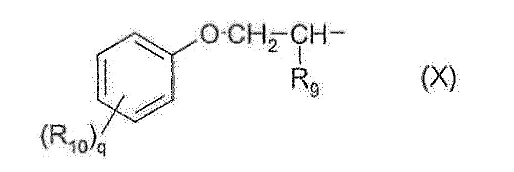 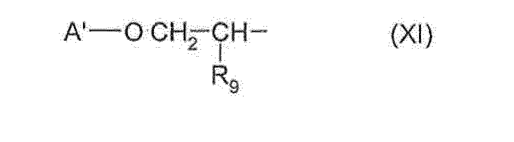 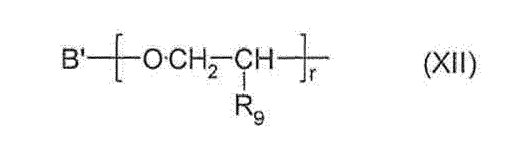
in which:
- R₉ is hydrogen or a methyl radical;
- R₁₀ is a C₁-C₉ alkyl radical;
- q is an integer ranging from 0 to 3;
- r is an integer ranging from 1 to 10;
- A' is a C₄-C₈ alkyl radical or a C₅-C₈ cycloalkyl radical;
- B' is chosen from: a linear or branched C₁C₈ alkyl radical; a C₅-C₈ cycloalkyl radical; an aryl radical optionally substituted with one or more C₁-C₄ alkyl radicals.

14. Composition according to Claim 13, in which the 1,3,5-triazine derivatives corresponding to formula (VIII) are chosen from those for which A₁,A₂ and A₃ are of formula (IX) and have the following characteristics:
- one of the radicals Xₐ-Rₐ represents a radical -NH-Rₐ with Rₐ chosen from: a C₅-C₁₂ cycloalkyl radical optionally substituted with one or more C₁-C₄ alkyl radicals; a radical of formula (X), (XI) or (XII) above in which B' is a C₁-C₄ alkyl radical and R₁₀ is a methyl radical;
- the other two radicals Xₐ-Rₐ represent a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which B' is a C₁-C₄ alkyl radical and R₁₀ is a methyl radical.

15. Composition according to Claim 13, in which the 1, 3, 5-triazine derivatives corresponding to formula (VIII) are chosen from those for which A₁, A₂ and A₃ are of formula (IX) and have the following characteristics:
- one or two groups Xₐ-Rₐ represent a radical -NH-Rₐ, with Rₐ chosen from: a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which B' is a C₁-C₄ alkyl radical and R₁₀is a methyl radicals;
- the other or the other two group (s) Xₐ-Rₐ being a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radicals of formula (X), (XI) or (XII) above in which B' is a C₁-C₄ alkyl radical and R₁₀ is a methyl radical.

16. Composition according to Claim 15, in which the 1, 3, 5-triazine derivative of formula (VIII) is 2-[(p-(tert-butylamido)anilino]-4,6-bis[(p-(2'-ethylhexyl-1'-oxycarbonyl)aniline]-1, 3, 5-triaz.ine corresponding to the following formula: 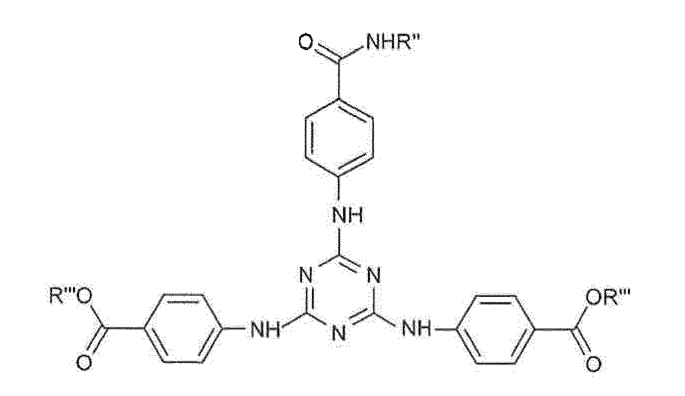 in which: R"' denotes a 2-ethylhexyl radical and R" denotes a tert-butyl radical.

17. Composition according to Claim 16, in which the 1, 3, 5-triazine derivatives corresponding to formula (VIII) are chosen from those for which A₁, A₂ and A₃ are of formula (IX) have following characteristics:
- Xₐ identical and represent oxygen;
- Rₐ, which: may be identical or different, represent a C₆-C₁₂ alkyl radical or a polyoxyethylene radial comprising from 1 to 6 ethylene oxide units and in which the terminal OH group is methylated.

18. Composition according to Claim 14, in which the 1,3,5-triazine derivative of formula (VIII) is 2,4,6-tris[p-(2'-ethylhexyl-l'-oxycarbonyl)anilino]-1,3,5-triazine corresponding to the following formula: 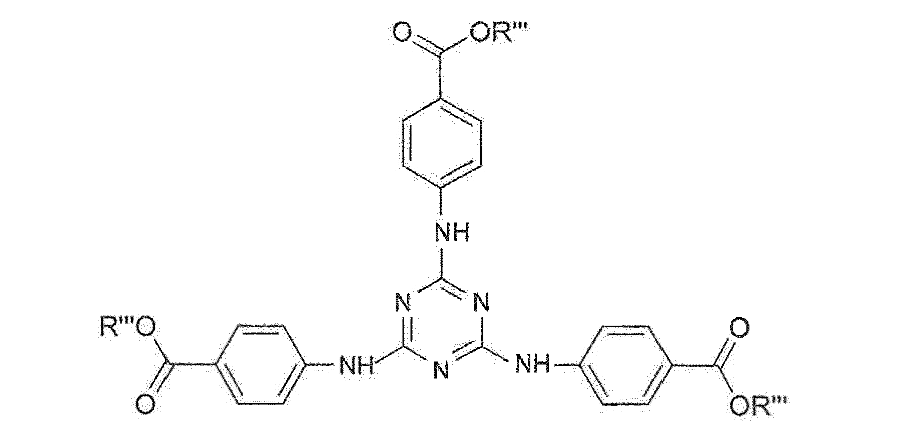 in which R'" denotes a 2-ethylhexyl radical.

19. Composition according to any one of Claims 1 to 18, in which the photosensitive 1,3,5-triazine compound(s) is (are) present in contents ranging from 0.01% to 20% by weight, more preferentially from 0.1% to 10% by weight and even more particularly from 0.1% to 6% by weight relative to the total weight of the composition.

20. Composition according to any one of the preceding claims, **characterized in that** it also contains other UVA-active and/or UVB-active organic or mineral photoprotective agents.

21. Composition according to Claim 11, in which the additional organic photoprotective agents are chosen from anthranilates; cinnamic derivatives; salicylic derivatives; camphor derivatives; benzophenone derivatives; β,β-diphenylacrylate derivatives; benzalmalonate derivatives; benzimidazole derivatives; imidazolines; bis-benzazolyl derivatives; p-aminobenzoic acid (PABA) derivatives; benzoxazole derivatives; screening polymers and screening silicones; α-alkylstyrene-based dimers; 4,4-diarylbutadienes, and mixtures thereof.

22. Composition according to Claim 19, **characterized in that** the organic UV-screening agent(s) is (are) chosen from the following compounds:
ethylhexyl methoxycinnamate,
Homosalate,
ethylhexyl salicylate,
Octocrylene,
phenylbenzimidazolesulfonic acid,
disodium phenyldibenzimidazoletetrasulfonate,
terephthalylidenedicamphorsulfonic acid, Benzophenone-3,
Benzophenone-4,
Benzophenone-5,
4-methylbenzylidenecamphor,
Polysilicone-15,
1,1-dicarboxy(2,2'-dimethylpropyl)-4,4-diphenylbutadiene,
and mixtures thereof.

23. Composition according to Claim 20, **characterized in that** the additional mineral photoprotective agents are treated or untreated metal oxide pigments.

24. Composition according to Claim 23, **characterized in that** the said pigments are chosen from treated or untreated titanium oxide, zinc oxide, iron oxide, zirconium oxide and cerium oxide, and mixtures thereof.

25. Composition according to any one of Claims 1 to 24, **characterized in that** it also comprises at least one agent for artificially tanning and/or browning the skin.

26. Composition according to any one of Claims 1 to 25, **characterized in that** it also comprises at least one adjuvant chosen from fatty substances, organic solvents, ionic or nonionic, hydrophilic or lipophilic thickeners, softeners, humectants, opacifiers, stabilizers, emollients, silicones, antifoams, fragrances, preserving agents, anionic, cationic, nonionic, zwitterionic or amphoteric surfactants, active agents, fillers, polymers, propellants, and acidifying or basifying agents.

27. Composition according to any one of Claims 1 to 26, **characterized in that** it is in the form of an oil-in-water or water-in-oil emulsion.

28. Use of a composition as defined in any one of Claims 1 to 27, for the manufacture of cosmetic products for treating the skin, the lips, the nails, the hair, the eyelashes, the eyebrows and/or the scalp.

29. Use of a composition as defined in any one of Claims 1 to 27, for the manufacture of products for caring for the skin, the lips, the nails, the hair and/or the scalp.

30. Use of a composition as defined in any one of Claims 1 to 27, for the manufacture of makeup products.

31. Process for improving the stability to UV radiation of a 1,3,5-triazine derivative that is photosensitive in the presence of a dibenzoylmethane derivative as defined in the preceding claims, **characterized in that** it consists in adding to the said combination at least one siliceous s-triazine compound substituted with two aminobenzoate or aminobenzamide groups of formula (I), or a tautomeric form thereof, as defined in the preceding claims.

32. Use of at least one siliceous s-triazine compound substituted with two aminobenzoate or aminobenzamide groups of formula (I), or a tautomeric form thereof, as defined in the preceding claims, in a composition containing at least one dibenzoylmethane derivative and at least one 1, 3, 5-triazine derivative that is photosensitive in the presence thereof, as defined in the preceding claims, for the purpose of improving the photostability of the said 1, 3, 5-triazine derivative.

## Patentansprüche

1. Zusammensetzung, die in einem physiologisch unbedenkliches Träger mindestens ein Filtersystem umfasst, **dadurch** gekenntzeichnet, dass diese Folgende umfaßt:
(a) mindestens ein UV-Filter des Dibenzoylmethanderivattyps und
(b) mindestens ein UV-Filter des lichtempfindlichen 1, 3, 5-Triazintyps in Gegenwart eines Dibenzoyimethanderivats und
(c) mindestens eine durch zwei Aminobenzoat- oder Aminobenzamidgruppen substituierte siliciumhaltige s-Triazinverbindung der Formel (I) unten oder eine ihrer tautomeren Formen: 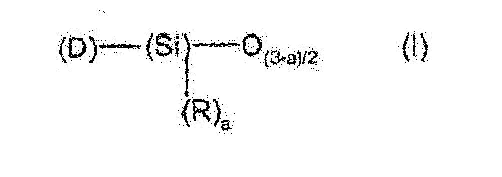 mit den folgenden Bedeutungen:
- R, die gleich oder verschieden sind, bedeuten einem geradkettigen oder verzweigten und gegebenenfalls halogenierten oder ungesättigten C₁-C₃₀-Alkylrest, einen C₆-C₁₂-Arylrest, einen C₁-C₁₀-Alkoxyrest oder die Trimethylsilyloxygruppe;
- a = 0 bis 3;
- die Gruppe D bedeutet eine s-Triazinverbindung der Formel (II) unten: 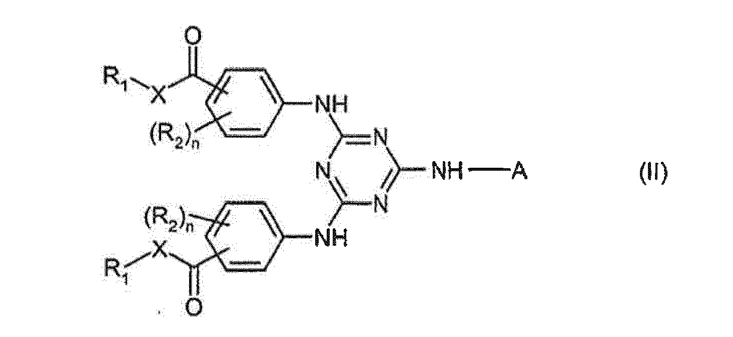
mit den folgenden Bedeutungen:
- X bedeutet -O- oder -NR₃-, wobei R₃ Wasserstoff oder einen C₁-C₅-Alkylrest bedeutet,
- R₁ bedeutet einen geradkettigen oder verzweigten und gegebenenfalls ungesättigten C₁-C₂₀-Alkylrest, der ein Siliciumatom enthalten kann, eine C₅-C₂₀-Cycloalkylgruppe, die gegebenenfalls durch 1 bis 3 geradkettige oder verzweigte C₁-C₄-Alkylreste substituiert ist, die Gruppe -(CH₂CHR₄-O)ₘR₅ oder die Gruppe -CH₂-CH(OH)-CH₂-O-R₆,
- R₄ bedeutet Wasserstoff oder Methyl; wobei die Gruppe (C=O)XR₁ in der ortho-, meta- oder para-Stellung der Aminogruppe stehen kann,
- R₅ bedeutet Wasserstoff oder eine C₁-C₈-Alkylgruppe,
- R₆ bedeutet Wasserstoff oder eine C₄-C₈-Alkylgruppe,
- m bedeutet eine ganze Zahl von 2 bis 20,
- n = 0 bis 2,
- R₂, die gleich oder verschieden sind, bedeuten einen Hydroxylrest, einen geradkettigen oder verzweigten C₁-C₈-Alkylrest, einen C₁-C₈-Alkoxyrest, wobei zwei benachbarte R₂ desselben aromatischen Rings gemeinsam eine Alkylidendioxygruppe, in der die Alkylidengruppe 1 oder 2 Kohlenstoffatome enthält, bilden können,
- A bedeutet einen zweiwertigen Rest aus der Gruppe Methylen, -[CH(Si(CH₃)₃)]-, Ethylen oder eine Gruppe gemäß einer der Formen (III), (IV) oder (V) unten: 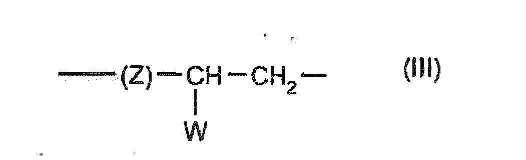 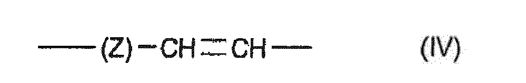 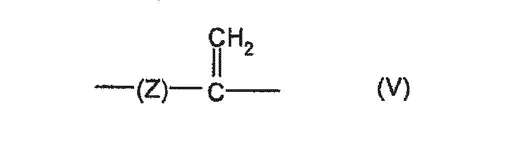
mit den folgenden Bedeutungen:
- Z bedeutet ein geradkettiges oder verzweigtes, gesättigtes oder ungesättigtes C₁-C₁₀-Alkylendiradikal, das gegebenenfalls durch einen Hydroxylrest oder durch Sauerstoffe substituiert ist und das gegebenenfalls eine Aminogruppe enthalten kann,
- W bedeutet ein Wasserstoffatom, einen Hydroxylrest oder einen geradkettigen oder verzweigten, gesättigten oder ungesättigten C₁-C₈-Alkylrest.

2. zuaammensetzung nach Anspruch 1, wobei die Verbindung der Formel (I) in der tautomeren Form der Formel (I') unten vorliegt: 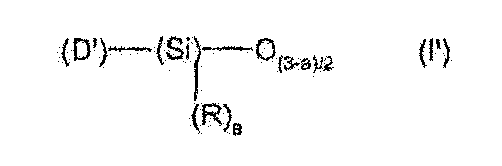 in der die Gruppe D' eine s-Triazinverbindung der Formel (II') unten bedeutet:

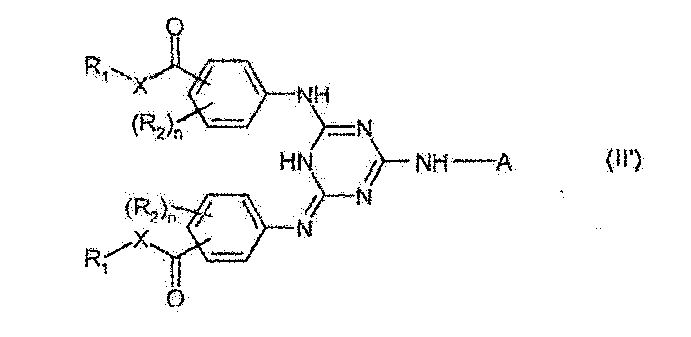

3. Zusammensetzung nach Anspruch 1 oder 2, wobei die Verbindung der Formel (I) zusätlich Einheilen der Formel (R)_{b}-(Si)(O)_{(4-b)/2} umfasst, in denen:
R die gleiche Bedeutung die in Formel (I) aufweist,
b = 1, 2 der 3.

4. Verbindung nach Anspruch 3, wobei die Verbindungen der Formel (I) aus denjenigen ausgewählt sind bei denen mindestens eine und noch stärker bevorzugt alle der folgenden Bedingunger verfüllt sind:
R bedeutet Methyl,
a = 1 oder 2,
X bedeutet O,
R₁ bedeutet einen C₄-C₅-Rest,
n = 0,
die Gruppe (C=O)XR₁ steht in der para-Stellung in Bezug auf Aminogruppe,
Z = -CH₂-,
W = H.

5. Zusammensetzung nach einem der Ansprüche 1 bis 4, wobei die Verbindungen der Formel (I) aus denjenigen ausgewählt sind, die einer der Formeln (Ia), (Ib) oder (Ic) unten entsprechen: 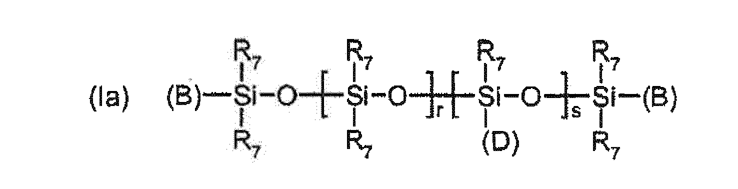 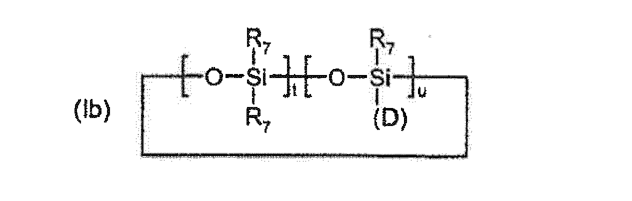
(Ic) (D)-Si(R₈)₃
mit den folgenden Bedeutungen:
- (D) entspricht der Formel (II) wie in den vorhergehenden Ansprüchen definiert,
- R₇, die gleich oder verschieden sind, sind ausgewählt aus der Reihe geradkettige oder verzweigte C₁-C₂₀-Alkylreste, Phenyl, 3,3,3-Trifluorpropyl und Trimethylsilyloxy oder dem Hydroxylrest,
- R₈, die gleich oder verschieden sind, sind ausgewählt aus der Reihe geradkettige oder verzweigte C₁-C₂₀-Alkyl- und Alkenylreste, Hydroxylrest oder Phenylrest,
- (B), die gleich oder verschieden sind, sind ausgewählt aus der Reihe R₇-Reste und (D)-Rest,
r bedeutet eine ganze Zahl zwischen einschließlich 0 und 200,
- s bedeutet eine ganze Zahl von 0 bis 50 und, wenn s = 0, so weist mindestens eines der beiden Symbole (B) die Bedeutung (D) auf,
- u bedeutet eine ganze Zahl von 1 bis 10,
- t bedeutet eine ganze Zahl von 0 bis 10, wobei gilt, dass t + u gleich oder größer 3 ist,
sowie ihre tautomeren Formen.

6. Zusammensetzung nach Anspruch 5, wobei die Verbindungen der Formel (Ia) oder (Ib) statistische Oligomere oder Polymers sind, die mindestens eine und noch stärker bevorzugt alle der folgenden Eigenschaften aufweisen:
- R₇ bedeutet den Methylrest oder den Hydroxylrest,
- B bedeutet vorzugsweise Methyl.

7. Zusammensetzung nach Anspruch, 6, wobei die Verbindung der Formel (I) aus der Reihe der Verbindungen der Formeln (a) bis (m) unten und ihren tautomeren Formen ausgewählt ist:

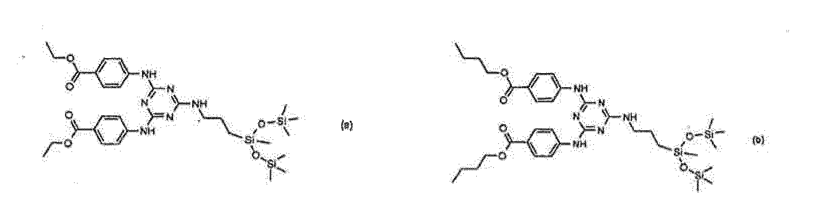

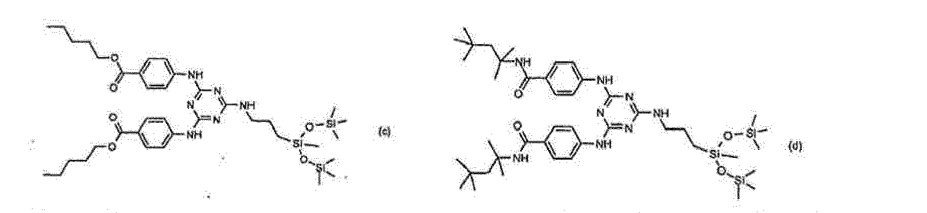

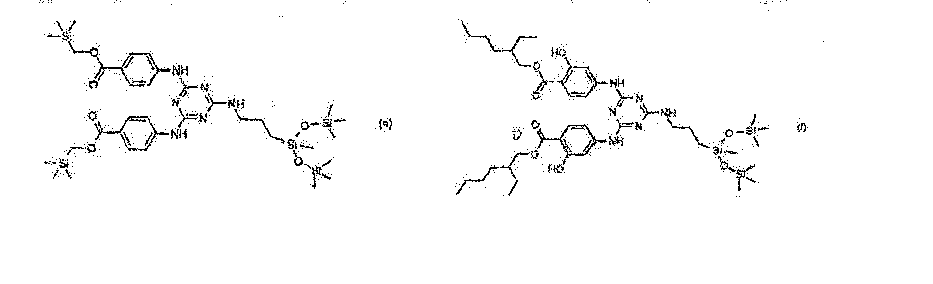

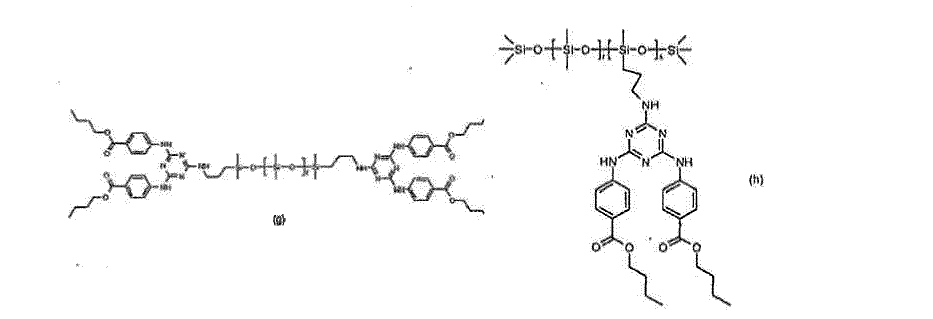

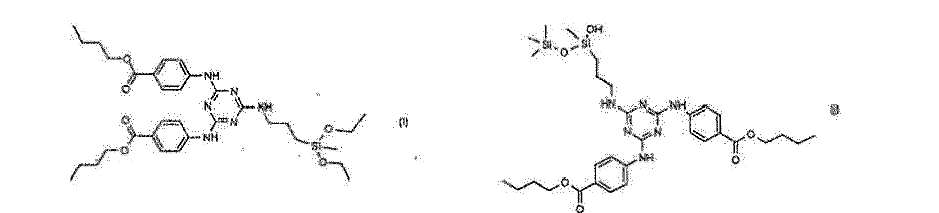

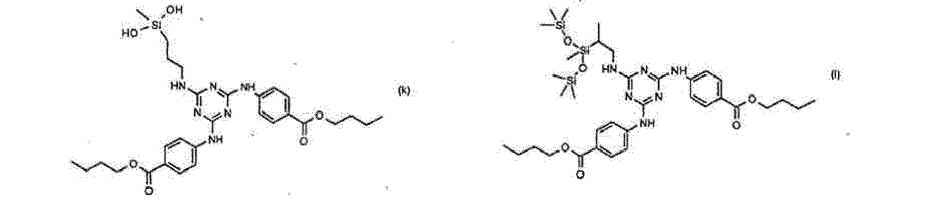

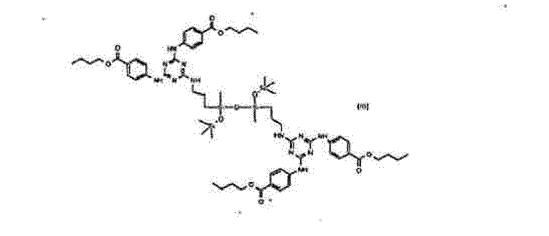

8. Zusammensetzung nach Anspruch 7, wobei es sich bei der Verbindung der Formel (I) um die Verbindung 2,4-Bis(n-butyl-4'-diylaminobenzoat)-6-{[1,3,3,3-tetramethyl-1-[(trimethylsilyl)oxy]disiloxanyl]propyl-3-ylamino}-s-triazin der Struktur (b) handelt:

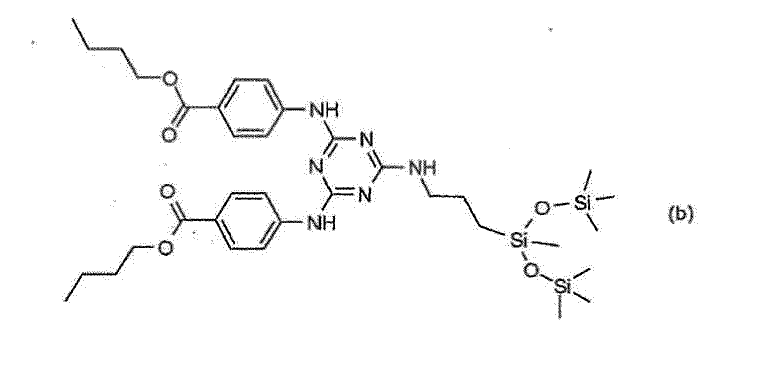

9. Zusammensetzung nach einem der vorhergehenden Ansprüche, wobei die s-Triazinverbindung(en) der Formel (I) in Gehalten von 0,01 bis 20 Gew.-%, stärker bevorzugt von 0,1 bis 10 Gew.-% und noch stärker bevorzugt 0,1 bis 6 Gew.-% in Bezug auf das Gesamtgewicht der Zusammensetzung vorliegt bzw. vorliegen.

10. Zusammensetzung nach einem der Ansprüche 1 bis 9, **dadurch gekennzeichnet, dass** das Dibenzoylmethanderivat der folgenden Gruppe ausgewählt ist:
- 2-Methyldibenzoylmethan
- 4-Methyldibenzoylmethan
- 4-Isopropyldibenzoylmethan
- 4-tert.-Butyldibenzoylmethan
- 2,4-Dimethyldibenzoylmethan
- 2,5-Dimethyldibenzoylmethan
- 4,4'-Diisopropyldibenzoylmethan
- 4,4'-Dimethoxydibenzoylmethan
- 4-tert.-Butyl-4'-methoxydibenzoylmethan
- 2-Methyl-5-isopropyl-4'-methoxydibenzoylmethan
- 2-Methyl-5-tert.-butyl-4'-methoxydibenzoylmethan
- 2,4-Dimethyl-4'-methoxydibenzoylmethan
- 2,6-Dimethyl-4-tert.-butyl-4'-methoxydibenzoylmethan.

11. Zusammensetzung nach Anspruch 10, **dadurch gekennzeichnet, dass** es sich bei dem Dibenzoylmethanderivat um 4-(tert.-Butyl)-4'-methoxydibenzoylmethan handelt.

12. Zusammensetzung nach einem der Ansprüche 1 bis 11, wobei das Dibenzoylmethanderivat in Gehalten von 0,01 bis 20 Gew.-%, stärker bevorzugt von 0,1 Gew.-% bis 10 Gew.-% und noch stärker besonders bevorzugt von 0,1 bis 6 Gew.-% in bezug auf das Gesamtgewicht der Zusammensetzung vorliegt.

13. Zusammensetzung nach einem der vorhergehenden Ansprüche, wobei das bzw. die UV-Filter des lipophilen nichtsiliciumhaltigen 1,3,5-Triazintyps aus der Reihe der 1,3,5-Triazinderivate der Formel (VIII) unten ausgewählt sind: 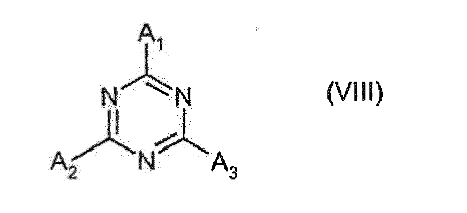 in der die Reste A₁, A₂ und A₃ gleich oder verschieden sind und aus der Reihe der Gruppen der Formeln (IX) ausgewählt sind: 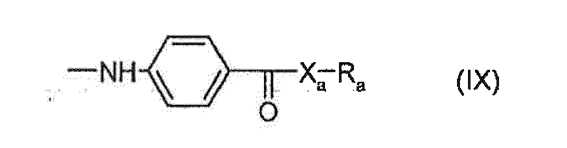 mit den folgenden Bedeutungen:
- Xₐ, die gleich oder verschieden sind, bedeuten Sauerstoff oder den -NH-Rest;
- Rₐ, die gleich oder verschieden sind, sind ausgewählt aus der Reihe: Wasserstoff; Alkalimetall;
gegebenenfalls durch einen oder mehrere Alkyl- oder Hydroxyalkylreste substituierter Ammoniumrest;
geradkettiger oder verzweigter C₁-C₁₈-Alkylrest; gegebenenfalls durch einen oder mehrere C₁-C₄-Alkylreste substituierter C₅-C₁₂-Cycloalkylrest; Polyoxyethylenrest, der 1 bis 6 Ethylenoxideinheiten umfasst, wobei die terminale OH-Gruppe methyliert ist; Rest der Formel (X), (XI) oder (XII) unten: 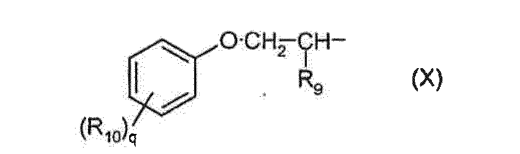 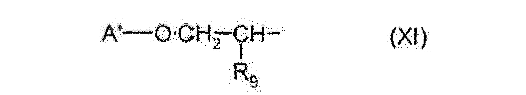 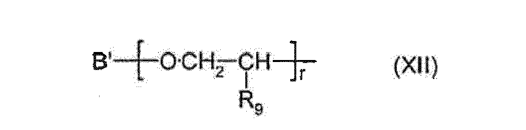 mit den folgenden Bedeutungen:
- R₉ bedeutet Wasserstoff oder einen Methylrest;
- R₁₀ bedeutet einen C₁-C₉-Alkylrest;
- q bedeutet eine ganze Zahl von 0 bis 3;
- r bedeutet eine ganze Zahl von 1 bis 10;
- A' bedeutet einen C₄-C₈-Alkylrest oder einen C₅-C₈-Cycloalkylrest;
- B' ist ausgewählt aus der Reihe: geradkettiger oder verzweigter C₁-C₈-Alkylrest; C₅-C₈-Cycloalkylrest;
Arylrest, der gegebenenfalls durch einen oder mehrere C₁-C₄-Alkylreste substituiert ist.

14. Zusammensetzung nach Anspruch 13, wobei die 1,3,5-Triazinderivate, die der Formel (VIII) entsprechen, aus denjenigen ausgewählt sind, bei denen A₁, A₂ und A₃ der Formel (IX) entsprechen und die die folgenden Eigenschaften aufweisen:
- einer der Reste Xₐ-Rₐ bedeutet den Rest -NH-Rₐ, wobei Rₐ aus Folgenden ausgewählt ist: einem C₅-C₁₂-Cycloalkylrest, der gegebenenfalls durch einen oder mehrere C₁-C₄-Alkylreste substituiert ist; einem Rest der Formel (X), (XI) oder (XII) oben, in denen B' einen C₁-C₄-Alkylrest bedeutet und R₁₀ den Methylrest bedeutet;
- die 2 anderen Xₐ-Rₐ bedeuten den Rest -O-Rₐ, wobei Rₐ, die gleich oder verschieden sind, aus Folgenden ausgewählt sind: Wasserstoff; einem Alkalimetall; einem gegebenenfalls durch einen oder mehrere Alkyl- oder Hydroxyalkylreste substituierten Ammoniumrest; einem geradkettigen oder verzweigten C₁-C₁₈-Alkylrest; einem C₅-C₁₂-Cycloalkylrest, der gegebenenfalls durch einen oder mehrere C₁-C₄-Alkylreste substituiert ist; einem Rest der Formel (X), (XI) oder (XII) oben, in denen B' einen C₁-C₄-Alkylrest bedeutet und R₁₀ den Methylrest bedeutet.

15. Zusammensetzung nach Anspruch 13, wobei die 1,3,5-Triazinderivate, die der Formel (VIII) entsprechen, aus denjenigen ausgewählt sind, bei denen A₁, A₂ und A₃ der Formel (IX) entsprechen und die die folgenden Eigenschaften aufweisen:
- einer oder beide der Reste Xₐ-Rₐ bedeutet bzw. bedeuten den Rest-NH-Rₐ, wobei Rₐ aus Folgenden ausgewählt ist: einem geradkettigen oder verzweigten C₁-C₁₈-Alkylrest; einem gegebenenfalls durch einen oder mehrere C₁-C₄-Alkylreste substituierten C₅-C₁₂-Cycloalkylrest; einem Rest der Formel (X), (XI) oder (XII) oben, in denen B' einen C₁-C₄-Alkylrest bedeutet und R₁₀ den Methylrest bedeutet;
- wobei der bzw. die zwei weitere (n) Rest(e) Xₐ-Rₐ den Rest -O-Rₐ bedeutet bzw, bedeuten, wobei Rₐ, die gleich oder verschieden sind, aus Folgenden ausgewählt sind: Wasserstoff; Alkalimetall; einem gegebenenfalls durch einen oder mehrere Alkyl- oder Hydroxyalkylreste substituierten Ammoniumrest; einem geradkettigen oder verzweigten C₁-C₁₈-Alkylrest; einem C₅-C₁₂-Cycloalkylrest, der gegebenenfalls durch einen oder mehrere C₁-C₄-Alkylreste substituiert ist; einem Rest der Formel (X), (XI) oder (XII) oben, in denen B' einen C₁-C₄-Alkylrest bedeutet und R₁₀ den Methylrest bedeutet.

16. Zusammensetzung nach Anspruch 15, wobei es sich bei dem 1,3,5-Triazinderivat der Formel (VIII) um 2-[(p-(tert.-Butylamido)anilino]-4,6-bis-[(p-(2'-ethylhexyl-1'-oxycarbonyl)anilino]-1,3,5-triazin der folgenden Formel handelt: 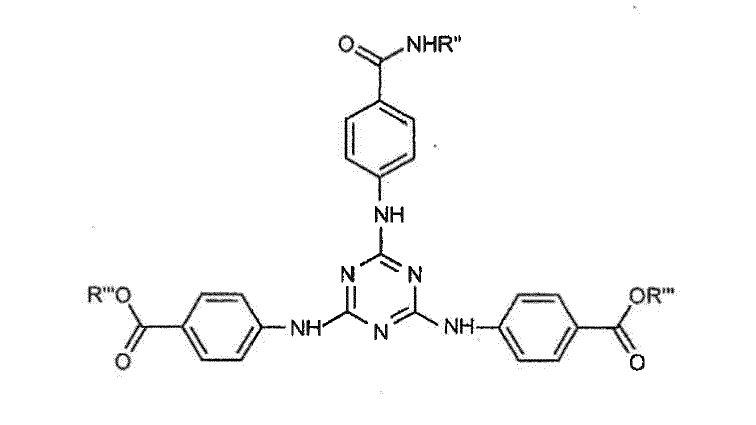 in der R"' einen 2-Ethylhexylrest bedeutet und R" einen tert.-Butylrest bedeutet.

17. Zusammensetzung nach Anspruch 16, wobei die 1,3,5-Triazinderivate, die der Formel (VIII) entsprechen, aus denjenigen ausgewählt sind, bei denen A₁, A₂ und A₃ der Formel (IX) entsprechen und die die folgenden Eigenschaften aufweisen:
- Xₐ sind gleich und bedeuten Sauerstoff;
- Rₐ, die gleich oder verschieden sind, entsprechen einem C₆-C₁₂-Alkylrest oder einem Polyoxyethylenrest mit 1 bis 6 Ethylenoxideinheiten, wobei die terminale OH-Gruppe methyliert ist.

18. Zusammensetzung nach Anspruch 14, wobei es sich bei dem 1,3,5-Triazinderivat der Formel (VIII) um 2,4,6-Tris[p-(2'-ethylhexyl-1'-oxycarbonyl)anilino]-1,3,5-triazin handelt, das der folgenden Formel entspricht: 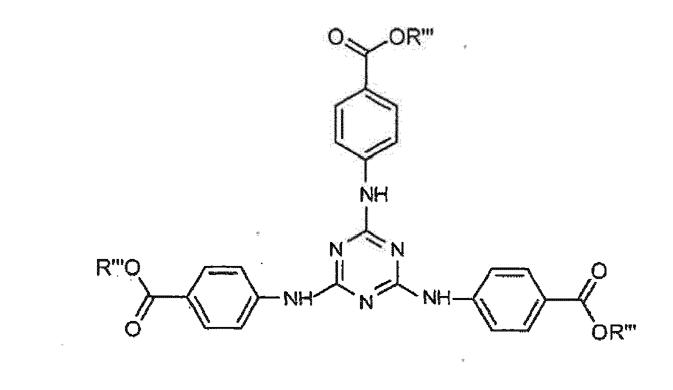 in der R"' einen 2-Ethylhexylrest bedeutet.

19. Zusammensetzung nach einem der Ansprüche 1 bis 18, wo die lichtempfindliche(n) 1,3,5-Triazinverbindung(en) in Gehalten von 0,01 bis 20 Gew.-%, stärker bevorzugt von 0,1 bis 10 Gew.-% und noch stärker besonders bevorzugt von 0,1 bis 6 Gew.-% in Bezug auf das Gesamtgewicht der Zusammensetzung vorhanden ist bzw. sind.

20. Zusammensetzung nach einem der vorhergehenden Ansprüche, **dadurch gekennzeichnet, dass** sie weiterhin andere organische oder anorganische Lichtschutzmittel, die im UV-A- und/oder UV-B-Bereich aktiv sind, enthält.

21. Zusammensetzung nach Anspruch 11, wobei die komplementären organischen Lichtschutzmittel aus der folgenden Gruppe ausgewählt sind: Anthranilate; Zimtsäurederivate; Salicylsäurederivate, Kampferderivate; Benzophenonderivate; β,β-Diphenylacrylatderivate; Benzalmalonatderivate; Benzimidazolderivate; Imidazoline; Bisbenzoazolylderivate; p-Aminobenzoesäurederivate (PABA-Derivate); Benzoxazolderivate; Filterpolymere und Filtersilikone; von α-Alkylstyrol abgeleitete Dimere; 4,4-Diarylbutadiene und ihre Mischungen.

22. Zusammensetzung nach Anspruch 19, **dadurch gekennzeichnet, dass** das bzw. die organische(n) UV-Filter aus der Reihe der folgenden Verbindungen ausgewählt ist bzw. sind:
Ethylhexylmethoxycinnamat
Homosalat
Ethylhexylsalicylat,
Octocrylen,
Phenylbenzimidazolsulfonsäure,
Dinatriumphenyldibenzimidazoltetrasulfonat,
Terephthalylidendikampfersulfonsäure
Benzophenon-3,
Benzophenon-4,
Benzophenon-5,
4-Methylbenzylidenkampfer,
Polysilikon-15
1,1-Dicarboxy(2,2'-dimethylpropyl)-4,4-diphenylbutadien und ihre Mischungen.

23. Zusammensetzung nach Anspruch 20, **dadurch gekennzeichnet, dass** es sich bei den komplementären anorganischen Lichtschutzmitteln um behandelte oder unbehandelte Metalloxidpigmente handelt.

24. Zusammensetzung nach Anspruch 23, **dadurch gekennzeichnet, dass** die Pigmente aus der Reihe Titanoxid, Zinkoxid, Eisenoxid, Zirkonoxid, Ceroxid und Mischungen davon, die behandelt oder unbehandelt sind, ausgewählt sind.

25. Zusammensetzung nach einem der Ansprüche 1 bis 24, **dadurch gekennzeichnet, dass** sie weiterhin mindestens ein Mittel für die künstliche Bräunung und/oder für die künstliche Braunfärbung der Haut enthält.

26. Zusammensetzung nach einem der Ansprüche 1 bis 25, **dadurch gekennzeichnet, dass** sie weiterhin mindestens einen Hilfsstoff aus der Reihe fettige Substanzen, organische Lösungsmittel, ionische oder nichtionische, hydrophile oder lipophile Verdickungsmittel, zartmachende Mittel, Feuchtigkeitsspender, Trübungsmittel, Stabilisatoren, Emollientien, Silikone, Schaumhemmer, Duftstoffe, Konservierungsmittel, anionische, kationische, nichtionische, zwitterionische oder amphotere Tenside, Wirkstoffe, Füllstoffe, Polymere, Treibmittel, Alkalinisierungsmittel oder Säuerungsmittel enthält.

27. Zusammensetzung nach einem der Ansprüche 1 bis 26, **dadurch gekennzeichnet, dass** sie in Form einer Öl-in-Wasser- oder Wasser-in-Öl-Emulsion vorliegt.

28. Verwendung einer Zusammensetzung wie in einem der Ansprüche 1 bis 27 definiert für die Herstellung von Produkten für die kosmetische Behandlung der Haut, der Lippen, der Nägel, des Haares, der Wimpern, der Augenbrauen und/oder der Kopfhaut.

29. Verwendung einer Zusammensetzung wie in einem der Ansprüche 1 bis 27 definiert für die Herstellung von Produkten für die Pflege der Haut, der Lippen, der Nägel, des Haares und/oder der Kopfhaut.

30. Verwendung einer Zusammensetzung wie in einem der Ansprüche 1 bis 27 definiert für die Herstellung von Make-up-Produkten.

31. Verfahren zum Verbessern der Stabilität eines lichtempfindlichen 1,3,5-Triazinderivats in Gegenwart eines Dibenzoylmethanderivats wie in einem der vorhergehenden Ansprüche definiert gegen UV-Strahlung, **dadurch gekennzeichnet, dass** es darin besteht, dass man diese Kombination mit mindestens einer durch zwei Aminobenzoat- oder Aminobenzamidgruppen substituierten siliciumhaltigen s-Triazinverbindung der Formel (I) oder einer ihrer tautomeren Formen wie in einem der vorhergehenden Ansprüche definiert versetzt.

32. Verwendung von mindestens einer durch zwei Aminobenzoat- oder Aminobenzamidgruppen substituierten siliciumhaltigen s-Triazinverbindung der Formel (I) oder einer ihrer tautomeren Formel wie in einem der vorhergehenden Ansprüche definiert in einer Zusammensetzung, die mindestens ein Dibenzoylmethanderivat und mindestens ein lichtempfindliches 1,3,5-Triazinderivat in Gegenwart von diesem wie in den vorhergehenden Ansprüchen definiert enthält, mit dem Zweck, die Lichtstabilität des 1,3,5-Triazinderivats zu verbessern.
